# EUROPEAN PATENT SPECIFICATION

(11) **EP 1 562 612 B1**
(45) Date of publication and mention of the grant of the patent: **27.01.2010**
(21) Application number: 03772404.4
(22) Date of filing: 07.11.2003
(51) Int. Cl.: A61K 31/70, A61K 31/517, A61K 45/00, A61P 35/00

(54) **COMBINATION PRODUCT OF INHIBITOR OF THE SRC FAMILY OF NON-RECEPTOR TYROSINE KINASES AND GEMCITABINE FOR USE IN THE TREATMENT OR PROPHLAXIX OF PANCREATIC CANCER**
KOMBINATIONSPRODUKT BESTEHEND AUS EINEM INHIBITOR DER SRC FAMILIE DER NICHT-REZEPTOR TYROSINKINASEN UND GEMCITABIN ZUR BEHANDLUNG ODER PROPHYLAXE VON PANKREASKREBS
PRODUIT COMBINANT UN INHIBITEUR DE LA FAMILLE SRC DES TYROSINE KINASES NON RECEPTRICES ET DE LA GEMCITABINE POUR LE TRAITEMENT DU CANCER PANCREATIQUE

(30) Priority: 13.11.2002 GB 0226434
(43) Date of publication of application: 17.08.2005
(73) Proprietor: AstraZeneca AB, 151 85 Södertälje (SE)
(72) Inventor: BARGE, Alan, Macclesfield, Cheshire SK10 4TG (GB)
(74) Representative: Tait, Brian Steele
(86) International application number: PCT/GB2003/004787
(87) International publication number: WO 2004/043472

(56) References cited:
- WO-A-02/057271
- MICHAEL M ET AL: "Clinical experience with gemcitabine in pancreatic carcinoma" ONCOLOGY, S. KARGER AG, BASEL, CH, vol. 11, no. 11, November 1997 (1997-11), pages 1615-1622, XP002117877 ISSN: 0030-2414
- LUTZ MANFRED P ET AL: "Overexpression and activation of the tyrosine kinase Src in human pancreatic carcinoma" BIOCHEMICAL AND BIOPHYSICAL RESEARCH COMMUNICATIONS, vol. 243, no. 2, 13 February 1998 (1998-02-13), pages 503-508, XP001180008 ISSN: 0006-291X cited in the application
- ITO HIROMICHI ET AL: "Inhibition of tyrosine kinase Src suppresses pancreatic cancer invasiveness." SURGERY (ST LOUIS), vol. 134, no. 2, August 2003 (2003-08), pages 221-226, XP009027101 ISSN: 0039-6060 (ISSN print)

## Description

The present invention relates to a combination comprising an inhibitor of the Src family of non-receptor tyrosine kinases, or a pharmaceutically acceptable salt thereof, wherein the Src inhibitor is:-
4-(2-chloro-5-methoxyanilino)-6-methoxy-7-(N-methylpiperidin-4-ylmethoxy)quinazoline, or a pharmaceutically-acceptable acid-addition salt thereof, or 4-(6-chloro-2,3-methylenedioxyanilino)-7-[2-(4-methylpiperazin-1-yl)ethoxy]-5-tetrahydropyran-4-yloxyquinazoline, or a pharmaceutically-acceptable acid-addition salt thereof, and gemcitabine for use in the synergistic treatment or prophylaxis of pancreatic cancer.

Current options for treating cancer include surgical resection, external beam radiation therapy and/or systemic chemotherapy. These are partially successful in some forms of cancer but are less successful in others. There is a clear need for new therapeutic treatments for treating cancer.

Many of the current treatment regimes for cell proliferation diseases such as cancer utilise compounds which inhibit DNA synthesis. Such compounds are toxic to cells generally but their toxic effect on rapidly dividing cells such as tumour cells can be beneficial. Alternative approaches to anti-tumour agents which act by mechanisms other than the inhibition of DNA synthesis have the potential to display enhanced selectivity of action.

In recent years it has been discovered that a cell may become cancerous by virtue of the transformation of a portion of its DNA into an oncogene *i.e.* a gene which, on activation, leads to the formation of malignant tumour cells (Bradshaw, Mutagenesis, 1986, 1, 91). Several such oncogenes give rise to the production of peptides which are receptors for growth factors. Activation of the growth factor receptor complex subsequently leads to an increase in cell proliferation. It is known, for example, that several oncogenes encode tyrosine kinase enzymes and that certain growth factor receptors are also tyrosine kinase enzymes (Yarden et al., Ann. Rev. Biochem., 1988, 57, 443; Larsen et al., Ann. Reports in Med. Chem, 1989, Chpt. 13). The first group of tyrosine kinases to be identified arose from such viral oncogenes, for example pp60^{v-Src} tyrosine kinase (otherwise known as v-Src), and the corresponding tyrosine kinases in normal cells, for example pp60^{c-Src} tyrosine kinase (otherwise known as c-Src).

Receptor tyrosine kinases are important in the transmission of biochemical signals which initiate cell replication. They are large enzymes which span the cell membrane and possess an extracellular binding domain for growth factors such as epidermal growth factor (EGF) and an intracellular portion which functions as a kinase to phosphorylate tyrosine amino acids in proteins and hence to influence cell proliferation. Various classes of receptor tyrosine kinases are known (Wilks, Advances in Cancer Research, 1993, 60, 43-73) based on families of growth factors which bind to different receptor tyrosine kinases. The classification includes Class I receptor tyrosine kinases comprising the EGF family of receptor tyrosine kinases such as the EGF, TGFα, Neu and erbB receptors, Class II receptor tyrosine kinases comprising the insulin family of receptor tyrosine kinases such as the insulin and IGF1 receptors and insulin-related receptor (IRR) and Class III receptor tyrosine kinases comprising the platelet-derived growth factor (PDGF) family of receptor tyrosine kinases such as the PDGFα, PDGFβ and colony-stimulating factor 1 (CSF1) receptors.

It is also known that certain tyrosine kinases belong to the class of non-receptor tyrosine kinases which are located intracellularly and are involved in the transmission of biochemical signals such as those that influence tumour cell motility, dissemination and invasiveness and subsequently metastatic tumour growth (Ullrich et al., Cell, 1990, 61, 203-212, Bolen et al., FASEB J., 1992, 6, 3403-3409, Brickell et al., Critical Reviews in Oncogenesis, 1992, 3, 401-406, Bohlen et al., Oncogene, 1993, 8, 2025-2031, Courtneidge et al., Semin. Cancer Biol., 1994, 5, 239-246, Lauffenburger et al., Cell, 1996, 84, 359-369, Hanks et al., BioEssays, 1996, 19, 137-145, Parsons et al., Current Opinion in Cell Biology, 1997, 9, 187-192, Brown et al., Biochimica et Biophysics Acta, 1996, 1287, 121-149 and Schlaepfer et al., Progress in Biophysics and Molecular Biology, 1999, 71, 435-478). Various classes of non-receptor tyrosine kinases are known including the Src family such as the Src, Lyn, Fyn and Yes tyrosine kinases, the Abl family such as Abl and Arg and the Jak family such as Jak 1 and Tyk 2.

It is known that the Src family of non-receptor tyrosine kinases are highly regulated in normal cells and in the absence of extracellular stimuli are maintained in an inactive conformation. However, some Src family members, for example c-Src tyrosine kinase, is frequently significantly activated (when compared to normal cell levels) in common human cancers such as gastrointestinal cancer, for example colon, rectal and stomach cancer (Cartwright et al., Proc. Natl. Acad. Sci. USA, 1990, 87, 558-562 and Mao et al., Oncogene, 1997, 15, 3083-3090), and breast cancer (Muthuswamy et al., Oncogene, 1995,11, 1801-1810). The Src family of non-receptor tyrosine kinases has also been located in other common human cancers such as non-small cell lung cancers (NSCLCs) including adenocarcinomas and squamous cell cancer of the lung (Mazurenko et al., European Journal of Cancer, 1992, 28, 372-7), bladder cancer (Fanning et al., Cancer Research, 1992, 52, 1457-62), oesophageal cancer (Jankowski et al., Gut, 1992, 33, 1033-8), cancer of the prostate, ovarian cancer (Wiener et al., Clin. Cancer Research, 1999, 5, 2164-70) and pancreatic cancer (Lutz et al., Biochem. and Biophys. Res. Comm. 1998, 243, 503-8). As further human tumour tissues are tested for the Src family of non-receptor tyrosine kinases it is expected that its widespread prevalence will be established.

It is further known that the predominant role of c-Src non-receptor tyrosine kinase is to regulate the assembly of focal adhesion complexes through interaction with a number of cytoplasmic proteins including, for example, focal adhesion kinase and paxillin. In addition c-Src is coupled to signalling pathways that regulate the actin cytoskeleton which facilitates cell motility. Likewise, important roles are played by the c-Src, c-Yes and c-Fyn non-receptor tyrosine kinases in integrin mediated signalling and in disrupting cadherin-dependent cell-cell junctions (Owens et al., Molecular Biology of the Cell, 2000, 11, 51-64 and Klinghoffer et al., EMBO Journal, 1999, 18, 2459-2471). Cellular motility is necessarily required for a localised tumour to progress through the stages of dissemination into the blood stream, invasion of other tissues and initiation of metastatic tumour growth. For example, colon tumour progression from localised to disseminated, invasive metastatic disease has been correlated with c-Src non-receptor tyrosine kinase activity (Brunton et al., Oncogene, 1997, 14, 283-293, Fincham et al., EMBO J, 1998, 17, 81-92 and Verbeek et al., Exp. Cell Research, 1999, 248, 531-537).

Accordingly it has been recognised that an inhibitor of such non-receptor tyrosine kinases should be of value as a selective inhibitor of the motility of tumour cells and as a selective inhibitor of the dissemination and invasiveness of mammalian cancer cells leading to inhibition of metastatic tumour growth. In particular an inhibitor of such non-receptor tyrosine kinases should be of value as an anti-invasive agent for use in the containment and/or treatment of solid tumour disease.

It is stated in International Patent Applications WO 01/94341 and WO 02/16352 that the Src inhibitors disclosed therein may be administered as a sole therapy or may involve, in addition to the quinazoline derivatives of those inventions, conventional surgery or radiotherapy or chemotherapy. Such chemotherapy was stated to include one or more of the following categories of anti-tumour agents :-
(i) other anti-invasion agents (for example metalloproteinase inhibitors like marimastat and inhibitors of urokinase plasminogen activator receptor function);
(ii) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea, or, for example, one of the preferred antimetabolites disclosed in European Patent Application No. 562734 such as (2S)-2- {o-fluoro-p-[N-{2,7-dimethyl-4-oxo-3,4-dihydroquinazolin-6-ylmethyl)-N-(prop-2-ynyl)amino]benzamido}-4-(tetrazol-5-yl)butyric acid); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(iii) cytostatic agents such as antioestrogens (for example tamoxifen, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrazole, vorazole and exemestane) and inhibitors of 5α-reductase such as finasteride;
(iv) inhibitors of growth factor function, for example such inhibitors include growth factor antibodies, growth factor receptor antibodies, tyrosine kinase inhibitors and serine/threonine kinase inhibitors, for example inhibitors of the epidermal growth factor family (for example the EGFR tyrosine kinase inhibitors N-(3-chloro-4-fluorophenyl)-7-methoxy-6-(3-morpholinopropoxy)quinazolin-4-amine (ZD1839), N-(3-ethynylphenyl)-6,7-bis(2-methoxyethoxy)quinazolin-4-amine (CP 358774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family; and
(v) antiangiogenic agents such as those which inhibit vascular endothelial growth factor such as the compounds disclosed in International Patent Applications WO 97/22596, WO 97/30035, WO 97/32856 and WO 98/13354 and those that work by other mechanisms (for example linomide, inhibitors of integrin αvβ3 function and angiostatin).

There is no specific disclosure of the combination use of a Src inhibitor and the antimetabolite cytotoxic agent gemcitabine, nor that any such combination produces surprisingly effective results.

We have unexpectedly found that a particular selection from the generic disclosures of combination therapies mentioned in International Patent Applications WO 01/94341 and WO 02/16352 is very effective. In particular, the combination of an inhibitor of the Src family of non-receptor tyrosine kinases, or a pharmaceutically-acceptable salt thereof, (referred to on occasions hereinafter as a Src inhibitor) and gemcitabine produces surprisingly effective results. More specifically, the combination of a Src inhibitor and gemcitabine produces a greater effect than that achievable by the administration of either a Src inhibitor alone or gemcitabine alone.

According to the present invention there is provided a combination comprising an inhibitor of the Src family of non-receptor tyrosine kinases, or a pharmaceutieally-acceptable salt thereof, wherein the Src inhibitor is:-
4-(2-chloro-5-methoxyanilino)-6-methoxy-7-(N-methylpiperidin-4-ylmethoxy)quinazoline, or a pharmaceutically-acceptable acid-addition salt thereof, or 4-(6-chloro-2,3-methylenedioxyanilino)-7-[2-(4-methylpiperazin-1-yl)ethoxy]-5-tetrahydropyran-4-yloxyquinazoline, or a pharmaceutically-acceptable acid-addition salt thereof, and gemcitabine for use in the synergistic treatment or prophylaxis of pancreatic cancer.

It is to be understood that term "a combination" envisages the simultaneous, sequential or separate administration of the components of the combination. In one aspect of the invention, "a combination" envisages simultaneous administration of the Src inhibitor and gemcitabine. In a further aspect of the invention, "a combination" envisages sequential administration of those agents. In another aspect of the invention, "a combination" envisages separate administration of those agents. Where the administration of those agents is sequential or separate, the delay in administering the second component should not be such as to lose the benefit of the synergistic effect of the combination therapy. Thus, for the avoidance of doubt, the present invention provides a combination comprising an inhibitor of the Src family of non-receptor tyrosine kinases, or a pharmaceutically-acceptable salt thereof, and gemcitabine for use simultaneously, sequentially or separately in the synergistic treatment or prophylaxis of cancer.

Suitable compounds possessing inhibitory activity against the Src family of non-receptor tyrosine kinases include the quinazoline derivatives disclosed in International Patent Applications WO 01/94341, WO 02/16352, WO 02/30924, WO 02/30926, WO 02/34744, WO 02/085895, WO 02/092577 (arising from PCT/GB 02/02117), WO 02/092578 (arising from PCT/GB 02/02124) and WO 02/092579 (arising from PCT/GB 02/02128), the quinoline derivatives described in WO 03/008409 (arising from PCT/GB 02/03177), WO 03/047584 and WO 03/048159 and the quinazoline derivatives described in European Patent Applications 02292736.2 (filed 04Nov2002) and 03290900.4 (filed 10Apr2003).

It is disclosed in Journal Medicinal Chemistry, 2001, 44, 822-833 and 3965-3977 that certain 4-anilino-3-cyanoquinoline derivatives are useful for the inhibition of Src-dependent cell proliferation. The 4-anilino-3-cyanoquinoline Src inhibitor known as SKI 606 is described in Cancer Research, 2003, 63, 375.

Other compounds which possess Src kinase inhibitory properties are described in, for example, International Patent Applications WO 96/10028, WO 97/07131, WO 97/08193, WO 97/16452, WO 97/28161, WO 97/32879 and WO 97/49706.

Other compounds which possess Src kinase inhibitory properties are described in, for example, International Patent Application WO 03/013540 [particularly the compounds disclosed therein by way of Formulae I to VIII and compounds of Formulae VII and VIII wherein the 2,6-dimethylphenyl group is replaced by a 2,6-dichlorophenyl or a 2-chloro-6-methylphenyl group].

Other compounds which possess Src kinase inhibitory properties are described in, for example, J Bone Mineral Research, 1999, 14 (Suppl. 1), S487, Molecular Cell, 1999, 3, 639-647, Journal Medicinal Chemistry, 1997, 40, 2296-2303, Journal Medicinal Chemistry, 1998, 41, 3276-3292 and Bioorganic & Medicinal Chemistry Letters, 2002, 12, 1361 and 3153.

Particular Src kinase inhibitors include :-
(i) 4-amino-5-(3-methoxyphenyl)-7-{4-[2-(2-methoxyethylamino)ethoxy]phenyl}-pyrrolo[2,3-*d*]pyrimidine and 4-amino-5-(3-methoxyphenyl)-7-(4-{2-[di-(2-methoxyethyl)amino]ethoxy}phenyl)pyrrolo[2,3-*d*]pyrimidine which are obtainable by methods described in International Patent Application WO 96/10028;
(ii) 4-amino-7-*tert*-butyl-5-(4-tolyl)pyrazolo[3,4-*d*]pyrimidine which is also known as PP1 and is described in Molecular Cell, 1999, 3, 639-648;
(iii) 2-(2,6-dichloroanilino)-6,7-dimethyl-1,8-dihydroimidazo[4,5-*h*]isoquinolin-9-one and 2-(2,6-dichloroanilino)-7-[(E)-3-diethylaminoprop-1-enyl]-6-methyl-1,8-dihydroimidazo[4,5-*h*]isoquinolin-9-one which are obtainable by methods described in Journal Medicinal Chemistry, 2002, 45, 3394;
(iv) 1-[6-(2,6-dichlorophenyl)-2-(4-diethylaminobutyl)pyrido[2,3-*d*]pyrimidin-7-yl]-3-ethylurea which is obtainable by methods described in Journal Medicinal Chemistry, 1997, 40, 2296-2303 and Journal Medicinal Chemistry. 2001, 44, 1915;
(v) 6-(2,6-dichlorophenyl)-2-[4-(2-diethylaminoethoxy)anilino]-8-methyl-8*H*-pyrido[2,3-*d*]pyrimidin-7-one which is also known as PD166285 and is described in J. Pharmacol. Exp. Ther., 1997, 283, 1433-1444;
(vi) the compound known as PD162531 which is described in Mol. Biol. Cell, 2000, 11, 51-64;
(vii) the compound known as PD166326 which is described in Biochem Pharmacol., 2000, 60, 885-898; and
(viii) the compound known as PD173955 which is described in Cancer Research, 1999, 59, 6145-6152.

Other compounds which may possess Src kinase inhibitory properties are described in, for example, International Patent Applications WO 02/079192, WO 03/000188, WO 03/000266, WO 03/000705, WO 02/083668, WO 02/092573, WO 03/004492, WO 00/49018, WO 03/013541, WO 01/00207, WO 01/00213 and WO 01/00214.

Particular Src inhibitors include the following compounds from International Patent Application WO 01/94341 :-
4-(2-chloro-5-methoxyanilino)-5,7-di-(3-morpholinopropoxy)quinazoline,
4-(2-bromo-5-methoxyanilino)-7-methoxy-5-(N-methylpiperidin-4-yloxy)quinazoline,
4-(2-chloro-5-methoxyanilino)-7-methoxy-5-(N-methylpiperidin-4-yloxy)quinazoline,
4-(2-chloro-5-methoxyanilino)-7-[3-(4-methylpiperazin-1-yl)propoxy]-5-tetrahydropyran-4-yloxyquinazoline,
4-(2-chloro-5-methoxyanilino)-7-(3-morpholinopropoxy)-5-tetrahydropyran-4-yloxyquinazoline,
4-(2-chloro-5-methoxyanilino)-7-[2-hydroxy-3-(4-methylpiperazin-1-yl)propoxy]-5-tetrahydropyran-4-yloxyquinazoline,
4-(2-chloro-5-methoxyanilino)-7-(2-hydroxy-3-morpholinopropoxy)-5-tetrahydropyran-4-yloxyquinazoline,
4-(2-chloro-5-methoxyanilino)-7-[3-(4-methylpiperazin-1-yl)propoxy]-5-tetrahydrofuran-3-yloxyquinazoline,
4-(2-chloro-5-methoxyanilino)-7-(3-morpholinopropoxy)-5-tetrahydrofuran-3-yloxyquinazoline,
4-(5-chloronaphth-1-ylamino)-7-methoxy-5-(N-methylpiperidin-4-yloxy)quinazoline,
4-(3-chlorobenzofuran-7-ylamino)-7-methoxy-5-(N-methylpiperidin-4-yloxy)quinazoline,
7-benzyloxy-4-(2-bromo-5-methoxyanilino)-5-piperidin-4-yloxyquinazoline,
4-(2-bromo-5-methoxyanilino)-7-(3-methylsulphonylpropoxy)-5-piperidin-4-yloxyquinazoline,
4-(2-bromo-5-methoxyanilino)-7-methoxy-5-piperidin-4-ylinethoxyquinazoline,
4-(2,4-dichloro-5-methoxyanilino)-7-methoxy-5-(N-methylpiperidin-4-yloxy)quinazoline,
4-(2,5-dimethoxyanilino)-7-methoxy-5-(N-methylpiperidin-4-yloxy)quinazoline,
4-(2,4-dichloro-5-methoxyanilino)-7-(2-pyrrolidin-1-ylethoxy)-5-tetrahydropyran-4-yloxyquinazoline,
4-(2,4-dichloro-5-methoxyanilino)-7-(2-piperidinoethoxy)-5-tetrahydropyran-4-yloxyquinazoline,
4-(2,4-dichloro-5-methoxyanilbo)-7-(2-morpholinoethoxy)-5-tetrahydropyrwx-4-yloxyquinazoline,
4-(2,4-dichloro-5-methoxyanilino)-7-[2-(4-methylpiperazin-1-yl)ethoxy]-5-tetrahydropyran-4-yloxyquinazoline,
4-(2-bromo-5-methoxyanilino)-7-(2-pyrrolidin-1-ylethoxy)-5-tetrahydropyran-4-yloxyquinazoline,
4-(2-bromo-5-methoxyanilino)-7-(2-piperidinoethoxy)-5-tetrahydropyran-4-yloxyquinazoline,
4-(2-bromo-5-methoxyanilino)-7-[2-(4-methylpiperazin-1-yl)ethoxy]-5-tetrahydropyran-4-yloxyquinazoline,
4-(2- bromo-5-methoxyanilino)-7-(4-pyridyloxyethoxy)-5-tetrahydropyran-4-yloxyquinazoline,
4-(2-bromo-5-methoxyanilino)-7-(2-[(2S)-2-(N,N-dimethylcarbamoyl)pyrrolidin-1-yl]ethoxy)-5-tetrahydropyran-4-yloxyquinazoline,
4-(2-bromo-5-methoxyanilino)-7-{2-[(2S)-2-(N-methylcarbamoyl)pyrrolidin-1-yl]ethoxy 5-tetrahydropyran-4-yloxyquinazoline,
4-(2-bromo-5-methoxyanilino)-7-(4-pyridylmethoxy)-5-tetrahydropyran-4-yloxyquinazoline,
4-(5-methoxy-2-pyrrolidin-1-ylanilino)-7-[3-(4-methylpiperazin-1-yl)propoxy]-5-tetrahydropyran-4-yloxyquinazoline,
4-(2-bromo-5-methoxyanilino)-5-cyclopentyloxy-7-(2-pyrrolidin-1-ylethoxy)quinazoline,
4-(6-chloro-2,3-methylenedioxyanilino)-5-cyclopentyloxy-7-(2-pyrrolidin-1-ylethoxy)quinazoline,
4-(6-chloro-2,3-methylenedioxyanilino)-5-piperidin-4-yloxyquinazoline,
4-(6-chloro-2,3-methylenedioxyanilino)-7-methoxy-5-piperidin-4-yloxyquinazoline,
4-(6-chloro-2,3-methylenedioxyanilino)-7-methoxy-5-(N-methylpiperidin-4-yloxy)quiuazoline,
4-(6-chloro-2,3-methylenedioxyanilino)-7-methoxy-5-piperidin-4-ylmethoxyquinazoline,
4-(6-chloro-2,3-methylenedioxyanilino)-7-(2-pyrrolidin-1-ylethoxy)-5-tetrahydropyran-4-yloxyquinazoline,
4-(6-chloro-2,3-methylenedioxyanilino)-7-(3-pyrrolidin-1-ylpropoxy)-5-tetrahydropyran-4-yloxyquinazoline,
4-(6-chloro-2,3-methylenedioxyanilino)-7-[3-(4-methylpiperazin-1-yl)propoxy]-5-tetrahydropyran-4-yloxyquinazoline,
4-(6-chloro-2,3-methylenedioxyanilino)-7-[2-(4-methylpiperazin-1-yl)ethoxy]-5-tetrahydropyran-4-yloxyquinazoline,
4-(6-chloro-2,3-methylenedioxyanilino)-7-(2-piperidinoethoxy)-5-tetrahydropyran-4-yloxyquinazoline,
4-(6-chloro-2,3-methylenedioxyanilino)-7-[2-(4-pyridyloxy)ethoxy]-5-tetrahydropyran-4-yloxyquinazoline,
4-(6-chloro-2,3-methylenedioxyanilino)-7-piperidin-4-ylmethoxy-5-tetrahydropyran-4-yloxyquinazoline and
4-(6-chloro-2,3-methylenedioxynilino)-7-(N-methylpiperidin-4-ylmethoxy)-5-tetrahydropyran-4-yloxyquinazoline;
   or a pharmaceutically-acceptable acid-addition salt thereof.

Further particular Src inhibitors include the following compounds from International Patent Application WO 02/16352 :-
6-methoxy-4-(2,3-methylenedioxyanilino)-7-(3-morpholinopropoxy)quinazoline,
6-methoxy-4-(2,3-methylenedioxyanilino)-7-[3-(1,1-dioxotetrahydro-4H-1,4-thiazin-4-yl)propoxy]quinazoline,
6-methoxy-4-(2,3-methylenedioxyanilino)-7-(3-pyrrolidin-1-ylpropoxy)quinazoline,
6-methoxy-4-(2,3-methylencdioxyanilino)-7-[2-(4-methylpiperazin-l-yl)ethoxy]quinazoline,
6-methoxy-4-(2,3-methylenedioxyanilino)-7-[3-(4-methylpiperazin-1-yl)propoxy]quinazoline,
6-methoxy-4-(2,3-methylenedioxyanilino)-7-(3-piperidinopropoxy)quinazoline,
6-methoxy-4-(2,3-methylenedioxyanilino)-7-(N-methylpiperidin-4-ylmethoxy)quinazoline,
7-(2-hydroxy-3-pyrrolidin-1-ylpropoxy)-6-methoxy-4-(2,3-methylenedioxyanilino)-quinazoline,
7-[2-hydroxy-3-(N-isopropyl-N-methylammo)propoxy]-6-methoxy-4-(2,3-methylenedioxyanilino)quinazoline,
7-[3-(4-cyanomethylpiperazin-1-yl)-2-hydroxypropoxy]-6-methoxy-4-(2,3-methylenedioxyanilino)quinazoline,
6-methoxy-4-(2,3-methylenedioxyanilino)-7-{2-[2-(4-methylpiperazin-1-yl)ethoxy]ethoxy}quinazoline,
4-(6-chloro-2,3-methylenedioxyanilino)-7-[3-(4-cyanomethylpiperazin-1-yl)propoxy]-6-methoxyquinazoline,
4-(6-chloro-2,3-methylenedioxyanilino)-6-methoxy-7-(3-pyrrolidin-1-ylpropoxy)quinazoline,
4-(6-chloro-2,3-methylenedioxyanilino)-6-methoxy-7-(3-piperidinopropoxy)quinazoline,
4-(6-bromo-2,3-methylenedioxyanilino)-6-methoxy-7-(3-piperidinopropoxy)quinazoline,
6-methoxy-4-(2,3-methylenedioxyanilino)-7-[2-(N-methylpiperidin-4-yl)ethoxy]quinazoline,
6-methoxy-4-(2,3-methylenedioxyanilino)-7-[2-(4-pyridyloxy)ethoxy]quinazoline,
6-methoxy-4-(2,3-methylenedioxyanilino)-7-(3-pyridylmethoxy)quinazoline,
4-(6-chloro-2,3-methylenedioxyanilino)-7-(2-cyanopyrid-4-ylmethoxy)-6-methoxyquinazoline and
4-(6-chloro-2,3-methylenedioxyanilino)-6-methoxy-7-(N-methylpiperidin-4-ylmethoxy)quinazoline;
or a pharmaceutically-acceptable acid-addition salt thereof.

Further particular Src inhibitors include the following compounds from International Patent Application WO 02/30924 :-
4-(7-benzofuranylamino)-6-methoxy-7-(3-morpholinopropoxy)quinazoline,
4-(7-benzofuranylamino)-6-methoxy-7-(3-pyrrolidin-1-ylpropoxy)quinazoline,
4-(7-benzofuranylamino)-6-methoxy-7-[3-(4-methylpiperazin-1-yl)propoxy]quinazoline,
4-(7-benzofuranylamino)-6-methoxy-7-(3-piperidinopropoxy)quinazoline,
4-(3-chlorobenzofuran-7-ylamino)-6-methoxy-7-(3-moipholinopropoxy)quinazoline,
4-(3-chlorobenzofuran-7-ylamino)-6-methoxy-7-(3-pyrrolidin-1-ylpropoxy)quinazoline,
4-(3-chlorobenzoruran-7-ylamino)-6-methoxy-7-[3-(4-methylpiperazin-1-yl)propoxy]quinazoline,
4-(3-chlorobenzofuran-7-ylamino)-6-methoxy-7-(3-piperidinopropoxy)quinazoline,
4-(6-chlorobenzofuran-7-ylatnino)-6-methoxy-7-(3-morpholinopropoxy)quinazoline,
4-(6-chlorobenzofuran-7-ylamino)-6-methoxy-7-(3-pyrrolidin-1-ylpropoxy)quinazoline,
4-(6-chlorobenzofuran-7-ylamino)-6-methoxy-7-[3-(4-methylpiperazin-1 - yl)propoxy]quinazoline,
4-(6-chlorobenzofuran-7-ylamino)-6-methoxy-7-(3-piperidinopropoxy)quinazoline,
4-(5-fluorobenzofuran-7-ylamino)-6-methoxy-7-(3-morpholinopropoxy)quinazoline,
4-(5-fluorobenzofuran-7-ylamino)-6-methoxy-7-(3-pyrrolidin-1-ylpropoxy)quinazoline,
4-(5-fluorobenzofuran-7-ylamino)-6-methoxy-7-[3-(4-methylpiperazin-1-yl)propoxy]quinazoline,
4-(5-fluorobenzofuran-7-ylamino)-6-methoxy-7-(3-piperidinopropoxy)quinazoline,
4-(7-benzofuranylamino)-6-methoxy-7-(N-methylpiperidin-4-ylmethoxy)quinazoline,
7-(2-acetoxy-3-pyrrolidin-1-ylpropoxy)-4-(3-chlorobenzofuran-7-ylamino)-6-methoxyquinazoline,
7-[2-acetoxy-3-(N-isopropyl-N-methylamino)propoxy]-4-(3-chlorobenzofuran-7-ylamino)-6-methoxyquinazoline,
7-[2-acetoxy-3-(4-cyanomethylpiperazin-1-yl)propoxy]-4-(3-chlorobenzofurau-7-ylamino)-6-methoxyquinazoline,
7-(2-acetoxy-3-piperidinopropoxy)-4-(3-chlorobenzofuran-7-ylamino)-6-methoxyquinazoline,
4-(3-chlorobenzofuran-7-ylamino)-7-(2-hydroxy-3-pyrrolidin-1-ylpropoxy)-6-methoxyquinazoline,
4-(3-chlorobenzofuran-7-ylamino)-7-[2-hydroxy-3-(N-isopropyl-N-methylamino)propoxy]-6-methoxyquinazoline,
4-(3-chlorobenzofuran-7-ylamino)-7-[3-(4-cyanomethylpiperazin-1-yl)-2-hydroxypropoxy]-6-methoxyquinazoline and
4-(3-chlorobenzofuran-7-ylamino)-7-(2-hydroxy-3-piperidinopropoxy)-6-methoxyquinazoline;
or a pharmaceutically-acceptable acid-addition salt thereof.

Further particular Src inhibitors include the following compounds from International Patent Application WO 02/30926 :-
4-(4-benzofuranylamino)-6-methoxy-7-(3-morpholinopropoxy)quinazoline,
4-(4-benzofuranylamino)-7-[3-(1,1-dioxotetrahydro-4H-1,4-thiazin-4-yl)propoxy]-6-methoxyquinazoline,
4-(4-benzofuranylamino)-6-methoxy-7-(3-pyrrolidin-1-ylpropoxy)quinazoline,
4-(4-benzofuranylamino)-6-methoxy-7-[3-(4-methylpiperazin-1-yl)propoxy]quinazoline,
4-(4-benzofuranylamino)-6-methoxy-7-(3-piperidinopropoxy)quinazoline,
4-(4-benzofuranylamino)-6-methoxy-7-(N-methylpiperidin-4-ylmethoxy)quinazoline,
4-(5-chlorobenzofuran-4-ylamino)-6-methoxy-7-(3-morpholinopropoxy)quinazoline,
7-(2-acetoxy-3-pyrrolidin-1-ylpropoxy)-4-(3-chlorobenzofuran-4-ylamino)-6-methoxyquinazoline,
7-[2-acetoxy-3-(N-isopropyl-N-methylatnino)propoxy]-4-(3-chlorobenzofuran-4-ylamino)-6-methoxyquinazoline,
7-[2-acetoxy-3-(4-cyanomethylpiperazin-1-yl)propoxy]-4-(3-chlorobenzofuran-4-ylamino)-6-methoxyquinazoline,
7-(2-acetoxy-3-piperidinopropoxy)-4-(3-chlorobenzofuran-4-ylamino)-6-methoxyquinazoline,
7-(2-acetoxy-3-morpholinopropoxy)-4-(3-chlorobenzofuran-4-ylamino)-6-methoxyquinazoline,
4-(4-benzofuranylamino)-7-(2-hydroxy-3-pyrrolidin-1-ylpropoxy)-6-methoxyquinazoline,
4-(4-benzofuranylamino)-7-[2-hydroxy-3-(N-isopropyl-N-methylamino)propoxy]-6-methoxyquinazoline,
4-(4-benzofuranylamino)-7-[3-(4-cyanomethylpiperazin-1-yl)-2-hydroxypropoxy]-6-methoxyquinazoline,
4-(4-benzofuranylamino)-7-(2-hydroxy-3-piperidinopropoxy)-6-methoxyquinazoline and
4-(4-benzofuranylamino)-7-(2-hydroxy-3-morpholinopropoxy)-6-methoxyquinazoline; or a pharmaceutically-acceptable acid-addition salt thereof.

Further particular Src inhibitors include the following compounds from International Patent Application WO 02/34744 :-
4-(7-indolylamino)-6-methoxy-7-(3-morpholinopropoxy)quinazoline,
4-(2,3-dimethylindol-7-ylamino)-6-methoxy-7-(3-morpholinopropoxy)quinazoline,
7-[3-(1,1-dioxotetrahydro-4H-1,4-thiazin-4-yl)propoxy]-4-(7-indolylamino)-6-methoxyquinazoline,
4-(7-indolylamino)-6-methoxy-7-(3-pyrrolidin-1-ylpropoxy)quinazoline,
4-(7-indolylanino)-6-methoxy-7-[3-(4-methylpiperazin-1-yl)propoxy]quinazoline,
4-(7-indolylamino)-6-methoxy-7-(3-piperidinopropoxy)quinazoline,
4-(3-chloroindol-7-ylamino)-6-methoxy-7-(3-morpholinopropoxy)quinazoline,
4-(7-indolylamino)-6-methoxy-7-(N-methylpiperidin-4-ylmethoxy)quinazoline,
7-(2-acetoxy-3-pyrrolidin-1-ylpropoxy)-4-(3-chloroindol-7-ylamino)-6-methoxyquiuazoline,
7-[2-acetoxy-3-(N-isopropyl-N-methylamino)propoxy]-4-(3-chloroindol-7-ylamino)-6-methoxyquinazoline,
7-[2-acetoxy-3-(4-cyanomethylpiperazin-1-yl)propoxy]-4-(3-chloroindol-7-ylamino)-6-methoxyquinazoline,
7-(2-acetoxy-3-piperidinopropoxy)-4-(3-chloroindol-7-ylamino)-6-methoxyquinazoline,
7-(2-acetoxy-3-morpholinopropoxy)-4-(3-chloroindol-7-ylamino)-6-methoxyquinazoline,
4-(3-chloroindol-7-ylamino)-7-(2-hydroxy-3-pyrrolidin-1-ylpropoxy)-6-methoxyquinazoline,
4-(3-chloroindol-7-ylamino)-7-[2-hydroxy-3-(N-isopropyl-N-methylamino)propoxy]-6-methoxyquinazoline,
4-(3-chloroindol-7-ylamino)-7-[3-(4-cyanomethylpiperazin-1-yl)-2-hydroxypropoxy]-6-methoxyquinazoline,
4-(3-chloroindol-7-ylamino)-7-(2-hydroxy-3-piperidinopropoxy)-6-methoxyquinazoline and
4-(3-chloroindol-7-ylamino)-7-(2-hydroxy-3-morpholinopropoxy)-6-methoxyquinazoline;
or a pharmaceutically-acceptable acid-addition salt thereof.

Further particular Src inhibitors include the following compounds from International Patent Application WO 02/085895 :-
6-methoxy-4-(2,3-methylenedioxyphenoxy)-7-(3-pyrrolidin-1-ylpropoxy)quinazoline,
4-(6-chloro-2,3-methylenedioxyphenoxy)-6-methoxy-7-(3-pyrrolidin-1-ylpropoxy)quinazoline,
4-(6-bromo-2,3-methylenedioxyphenoxy)-6-methoxy-7-(3-pyrrolidin-1-ylpropoxy)quinazoline,
6-methoxy-4-(2,3-methylenedioxyphenoxy)-7-(3-morpholinopropoxy)quinazoline,
4-(6-chloro-2,3-methylenedioxyphenoxy)-6-methoxy-7-(3-morpholinopropoxy)quinazoline,
4-(6-bromo-2,3-methylenedioxyphenoxy)-6-methoxy-7-(3-morpholinopropoxy)quinazoline,
6-methoxy-4-(2,3-methylenedioxyphenoxy)-7-[3-(4-methylpiperazin-1-yl)propoxy]quinazoline,
4-(6-chloro-2,3-methylenedioxyphenoxy)-6-methoxy-7-[3-(4-methylpiperazin-1-yl)propoxy]quinazoline,
4-(6-bromo-2,3-methylenedioxyphenoxy)-6-methoxy-7-[3-(4-methylpiperazin-1-yl)propoxy]quinazoline,
6-methoxy-4-(2,3-methylenedioxyphenoxy)-7-(3-methylsulphonylpropoxy)quinazoline,
4-(6-chloro-2,3-methylenedioxyphenoxy)-6-methoxy-7-(3-methylsulphonylpropoxy)quinazoline and
4-(6-bromo-2,3-methylenedioxyphenoxy)-6-methoxy-7-(3-methylsulphonylpropoxy)quinazoline;
or a pharmaceutically-acceptable acid-addition salt thereof.

Further particular Src inhibitors include the following compounds from International Patent Application WO 02/092579 (arising from PCT/GB 02/02117) :-
4-(2-chloro-5-methoxyanilino)-6-methoxy-7-(N-methylpiperidin-4-ylmethoxy)quinazoline,
4-(2-chloro-5-methoxyanilino)-6-methoxy-7-piperidin-4-ylmethoxyquinazoline and
4-(2-bromo-5-methoxyanilino)-6-methoxy-7-[2-(N-methylpiperidin-4-yl)ethoxy]quinazoline;
or a pharmaceutically-acceptable acid-addition salt thereof.

Further particular Src inhibitors include the following compounds from International Patent Application WO 02/092578 (arising from PCT/GB 02/02124) :-
4-(2,4-dichloro-5-methoxyanilino)-6-methoxy-7-(N-methylpiperidin-4-ylmethoxy)quinazoline,
4-(2,4-dichloro-5-methoxyanilino)-6-methoxy-7-piperidin-4-ylmethoxyquinazoline,
4-(2,4-dichloro-5-methoxyanilino)-6-methoxy-7-[2-(N-methylpiperidin-4-yl)ethoxy]quinazoline and
4-(2,4-dichloro-5-methoxyanilino)-6-methoxy-7-(2-piperidin-4-ylethoxy)quinazoline;
or a pharmaceutically-acceptable acid-addition salt thereof.

Further particular Src inhibitors include the following compounds from International Patent Application WO 02/092579 (arising from PCT/GB 02/02128) :-
4-(2-chloro-5-methoxyanilino)-6-methoxy-7-[3-(4-methylpiperazin-1-yl)propoxy]quinazoline,
4-(2-chloro-5-methoxyanilino)-6-methoxy-7-(2-piperidinoethoxy)quinazoline and
4-(2-chloro-5-methoxyanilino)-6-methoxy-7-(2-morpholinoethoxy)quinazoline and
4-(2-bromo-5-methoxyanilino)-6-methoxy-7-[3-(4-methylpiperazin-1-yl)propoxy]quinazoline
or a pharmaceutically-acceptable acid-addition salt thereof.

Further particular Src inhibitors include the following compounds from International Patent Application WO 03/008409 (arising from PCT/GB 02/03177) :-
4-(6-chloro-2,3-methylenedioxyanilino)-3-cyano-6-methoxy-7-[3-(4-methylpiperazin-1-yl)propoxy]quinoline,
4-(6-chloro-2,3-methylenedioxyanitino)-7-(3-chloropropoxy)-3-cyano-6-methoxyquinoline,
4-(6-chloro-2,3-methylenedioxyanilino)-3-cyano-7-methoxy-5-(N-methylpiperidin-4-yloxy)quinoline,
4-(6-chloro-2,3-methylenedioxyanilino)-3-cyano-7-(2-pyrrolidin-1-ylethoxy)-5-tetrahydropyran-4-yloxyquinoline,
4-(6-chloro-2,3-methylenedioxyanilino)-3-cyano-7-(3-pyrrolidin-1-ylpropoxy)-5-tetrahydropyran-4-yloxyquinoline,
4-(6-chloro-2,3-methylenedioxyanilino)-3-cyano-7-[3-(4-methylpiperazin-1-yl)propoxy]-5-tetrahydropyran-4-yloxyquinoline,
4-(6-chloro-2,3-methylenedioxyanilino)-3-cyano-7-[2-(4-methylpiperazin-1-yl)ethoxy]-5-tetrahydropyran-4-yloxyquinoline,
4-(6-chloro-2,3-methylenedioxyanilino)-3-cyano-7-(2-piperidinoethoxy)-5-tetrahydropyran-4-yloxyquinoline and
4-(6-chloro-2,3-methylenedioxyanilino)-3-cyano-7-(N-methylpiperidin-4-ylmethoxy)-5-tetrahydropyran-4-yloxyquinoline;
or a pharmaceutically-acceptable acid-addition salt thereof.

Further particular Src inhibitors include the following compounds from European Patent Applications 02292736.2 and 03290900.4 and as described in the Examples hereinafter :-
4-(5-chloro-2,3-methylenedioxypyrid-4-ylamino)-6-methoxy-7-[3-(4-prop-2-ynylpiperazin-1-yl)propoxy]quinazoline,
4-(5-chloro-2,3-methylenedioxypyrid-4-ylamino)-7-[3-(4-isobutyrylpiperazin-1-yl)propoxy]-6-methoxyquinazoline,
4-(5-chloro-2,3-methylenedioxypyrid-4-ylamino)-6-methoxy-7-{3-[4-(2,2,2-trifluoroethyl)piperazin-1-yl]propoxy}quinazoline,
4-(5-chloro-2,3-methylenedioxypyrid-4-ylamino)-6-methoxy-7-[2-(4-prop-2-ynylpiperazin-1-yl)ethoxy]quinazoline,
7-[2-(4-acetylpiperazin-1-yl)ethoxy]-4-(5-chloro-2,3-methylenedioxypyrid-4-ylamino)-5-tetrahydropyran-4-yloxyquinazoline,
4-(5-chloro-2,3-methylenedioxypyrid-4-ylamino)-7-{2-[(3RS,4SR)-3,4-methylenedioxypyrrolidin-1-yl]ethoxy}-5-tetrahydropyran-4-yloxyquinazoline,
4-(5-chloro-2,3-methylenedioxypyrid-4-ylamino)-7-[2-(4-prop-2-ynylpiperazin-1-yl)ethoxy]-5-tetrahydropyran-4-yloxyquinazoline,
4-(5-chloro-2,3-methylenedioxypyrid-4-ylamino)-7-[3-(4-prop-2-ynylpiperazin-1-yl)propoxy]-5-tetrahydropyran-4-yloxyquinazoline,
4-(5-chloro-2,3-methylenedioxypyrid-4-ylamino)-7-(2-morpholinoethoxy)-5-tetrahydropyran-4-yloxyquinazoline,
4-(5-chloro-2,3-methylenedioxypyrid-4-ylamino)-7-(3-morpholinopropoxy)-5-tetrahydropyran-4-yloxyquinazoline,
7-[2-(4-acetylpiperazin-1-yl)ethoxy]-4-(5-chloro-2,3-methylenedioxypyrid-4-ylamino)-5-isopropoxyquinazoline,
4-(5-chloro-2,3-methylenedioxypyrid-4-ylamino)-5-isopropoxy-7-(2-piperazin-1-ylethoxy)quinazoline,
4-(5-chloro-2,3-methylenedioxypyrid-4-ylamino)-7-{2-[4-(2-hydroxyethyl)piperazin-1-yl]ethoxy}-5-isopropoxyqumazoime,
4-(5-chloro-2,3-methylenedioxypyrid-4-ylamino)-5-isopropoxy-7-(2-pyrrolidin-1-ylethoxy)quinazoline,
4-(5-chloro-2,3-methylenedioxypyrid-4-ylamino)-5-isopropoxy-7-(2-piperidinoethoxy)quinazoline,
4-(5-chloro-2,3-methylenedioxypyrid-4-ylamino)-5-isopropoxy-7-(2-morpholinoethoxy)quinazoline,
4-(5-chloro-2,3-methylenedioxypyrid-4-ylamino)-5-isopropoxy-7-(3-morpholinopropoxy)quinazoline,
4-(5-chloro-2,3-methylenedioxypyrid-4-ylamino)-5-isopropoxy-7-[2-(4-prop-2-ynylpiperazin-1-yl)ethoxy]quinazoline,
4-(5-chloro-2,3-methylenedioxypyrid-4-ylamino)-5-isopropoxy-7-[2-(4-methylpiperazin-1-yl)ethoxy]quinazoline and
4-(5-chloro-2,3-methylenedioxypyrid-4-ylamino)-7-{2-[4-(2-dimethylaminoacetyl)piperazin-1-yl]ethoxy}-5-isopropoxyquinazoline;
or a pharmaceutically-acceptable acid-addition salt thereof.

More particular Src inhibitors include the following compounds :-
4-(2,4-dichloro-5-methoxyanilino)-7-(2-piperidinoethoxy)-5-tetrahydropyran-4-yloxyquinazoline,
4-(2,4-dichloro-5-methoxyanilino)-7-(2-morpholinoethoxy)-5-tetrahydropyran-4-yloxyquinazoline,
4-(2,4-dichloro-5-methoxyanilino)-7-[2-(4-methylpiperazin-1-yl)ethoxy]-5-tetrahydropyran-4-yloxyquinazoline,
4-(2-bromo-5-methoxyanilino)-7-(2-pyrrolidin-1-ylethoxy)-5-tetrahydropyran-4-yloxyquinazoline,
4-(6-chloro-2,3-methylenedioxyanilino)-7-(2-pyrrolidin-1-ylethoxy)-5-tetrahydropyran-4-yloxyquinazoline,
4-(6-chloro-2,3-methylenedioxyanilino)-7-(3-pyrrolidin-1-ylpropoxy)-5-tetrahydropyran-4-yloxyquinazoline,
4-(6-chloro-2,3-methylenedioxyanilino)-7-[3-(4-methylpiperazin-1-yl)propoxy]-5-tetrahydropyran-4-yloxyquinazoline,
4-(6-chloro-2,3-methylenedioxyanilino)-7-[2-(4-methylpiperazin-1-yl)ethoxy]-5-tetrahydropyran-4-yloxyquinazoline,
4-(6-chloro-2,3-methylenedioxyanilino)-7-(2-piperidinoethoxy)-5-tetrahydropyran-4-yloxyquinazoline,
6-methoxy-4-(2,3-methylenedioxyanilino)-7-(3-morpholinopropoxy)quinazoline,
6-methoxy-4-(2,3-methylenedioxyanilino)-7-[3-(1,1-dioxotetrahydro-4H-1,4-thiazin-4-yl)propoxy]quinazoline,
6-methoxy-4-(2,3-methylenedioxyanilino)-7-(3-pyrrolidin-1-ylpropoxy)quinazoline,
6-methoxy-4-(2,3-methylenedioxyanilino)-7-[2-(4-methylpiperazin-1-yl)ethoxy]quinazoline,
6-methoxy-4-(2, 3-methylenedioxyanilino)-7-[3-(4-methylpiperazin-1-yl)propoxy] quinazoline,
6-methoxy-4-(2,3-methylenedioxyanilino)-7-(3-piperidinopropoxy)quinazoline,
4-(2-chloro-5-methoxyanilino)-6-methoxy-7-(N-methylpiperidin-4-ylmethoxy)quinazoline,
4-(2-chloro-5-methoxyanilino)-6-methoxy-7-piperidin-4-ylmethoxyquinazoline,
4-(2-bromo-5-methoxyanilino)-6-methoxy-7-[2-(N-methylpiperidin-4-yl)ethoxy]quinazoline,
4-(2,4-dichloro-5-methoxyanilino)-6-methoxy-7-(N-methylpiperidin-4-ylmethoxy)quinazoline,
4-(2,4-dichloro-5-methoxyanilino)-6-methoxy-7-piperidin-4-ylmethoxyquinazoline and
4-(2,4-dichloro-5-methoxyanilino)-6-methoxy-7-[2-(N-methylpiperidin-4-yl)ethoxy]quinazoline;
or a pharmaceutically-acceptable acid-addition salt thereof.

Preferred Src inhibitors include the following compounds :-
4-(2,4-dichloro-5-methoxyanilino)-7-(2-piperidinoethoxy)-5-tetrahydropyran-4-yloxyquinazoline,
4-(2,4-dichloro-5-methoxyanilino)-7-(2-morpholinoethoxy)-5-tetrahydropyran-4-yloxyquinazoline,
4-(6-chloro-2,3-methylenedioxyanilino)-7-(2-pyrrolidin-1-ylethoxy)-5-tetrahydropyran-4-yloxyquinazoline,
4-(6-chloro-2,3-methylenedioxyanilino)-7-[2-(4-methylpiperazin-1-yl)ethoxy]-5-tetrahydropyran-4-yloxyquinazoline,
4-(6-chloro-2,3-methylenedioxyanilino)-7-(2-piperidmoethoxy)-5-tetrahydropyran-4-yloxyquinazoline,
6-methoxy-4-(2,3-methylenedioxyanilino)-7-(3-morpholinopropoxy)quinazoline,
6-methoxy-4-(2,3-methylenedioxyanilino)-7-(3-pyrrolidin-1-ylpropoxy)quinazoline,
6-methoxy-4-(2,3-methylenedioxyanilino)-7-[3-(4-methylpiperazin-1-yl)propoxy]quinazoline,
6-methoxy-4-(2,3-methylenedioxyanilino)-7-(3-piperidinopropoxy)quinazoline,
4-(2-chloro-5-methoxyanilino)-6-methoxy-7-(3-methylpiperidin-4-ylmethoxy)quinazoline,
4-(2-chloro-5-methoxyanilino)-6-methoxy-7-piperidin-4-ylmethoxyquinazoline,
4-(2,4-dichloro-5-methoxyanilino)-6-methoxy-7-(N-methylpiperidin-4-ylmethoxy)quinazoline and
4-(2,4-dichloro-5-methoxyanilino)-6-methoxy-7-piperidin-4-ylmethoxyquinazoline;
or a pharmaceutically-acceptable acid-addition salt thereof.

Further preferred Src inhibitors include the following compounds :-
4-(5-chloro-2,3-methylenedioxypyrid-4-ylamino)-6-methoxy-7-[3-(4-prop-2-ynylpiperazin-1-yl)propoxy]quinazoline,
7-[2-(4-acetylpiperazin-1-yl)ethoxy]-4-(5-chloro-2,3-methylenedioxypyrid-4-ylamino)-5-tetrahydropyran-4-yloxyquinazoline,
4-(5-chloro-2,3-methylenedioxypyrid-4-ylamino)-7-[3-(4-prop-2-ynylpiperazin-1-yl)propoxy]-5-tetrahydropyran-4-yloxyquinazoline,
4-(5-chloro-2,3-methylenedioxypyrid-4-ylamino)-7-(3-morpholinopropoxy)-5-tetrahydropyran-4-yloxyquinazoline,
7-[2-(4-acetylpiperazin-1-yl)ethoxy]-4-(5-chloro-2,3-methylenedioxypyrid-4-ylamino)-5-isopropoxyquinazoline,
4-(5-chloro-2,3-methylenedioxypyrid-4-ylamino)-5-isopropoxy-7-(2-piperazin-1-ylethoxy)quinazoline and
4-(5-chloro-2,3-methylenedioxypyrid-4-ylamino)-7-{2-[4-(2-hydroxyethyl)piperazin-1-yl]ethoxy}-5-isopropoxyquinazoline;
or a pharmaceutically-acceptable acid-addition salt thereof.

A particular preferred Src inhibitor for use in the combination of the invention is :-4-(6-chloro-2,3-methylenedioxyanilino)-7-(2-pyrrolidin-1-ylethoxy)-5-tetrahydropyran-4-yloxyquinazoline;
or a pharmaceutically-acceptable acid-addition salt thereof.

A further particular preferred Src inhibitor for use in the combination of the invention is:-
4-(6-chloro-2,3-methylenedioxyanilino)-7-[2-(4-methylpiperazin-1-yl)ethoxy]-5-tetrahydropyran-4-yloxyquinazoline;
or a pharmaceutically-acceptable acid-addition salt thereof.

A further particular preferred Src inhibitor for use in the combination of the invention is:-
4-(6-chloro-2,3-methylenedioxyanilino)-7-(2-piperidinoethoxy)-5-tetrahydropyran-4-yloxyquinazoline;
or a pharmaceutically-acceptable acid-addition salt thereof.

A further particular preferred Src inhibitor for use in the combination of the invention is:-
6-methoxy-4-(2,3-methylenedioxyanilino)-7-(3-morpholinopropoxy)quinazoline;
or a pharmaceutically-acceptable acid-addition salt thereof.

A further particular preferred Src inhibitor for use in the combination of the invention is:-
4-(2-chloro-5-methoxyanihno)-6-methoxy-7-(N-methylpiperidin-4-ylmethoxy)quinazoline;
or a pharmaceutically-acceptable acid-addition salt thereof.

A suitable pharmaceutically-acceptable salt of a Src inhibitor that is sufficiently basic is, for example, a pharmaceutically-acceptable acid-addition salt, for example an acid-addition salt with an inorganic or organic acid such as hydrochloric, hydrobromic, sulphuric, trifluoroacetic, citric or maleic acid. A suitable pharmaceutically-acceptable salt of a Src inhibitor that is sufficiently acidic is, for example, a pharmaceutically-acceptable alkali or alkaline earth metal salt such as a calcium or magnesium salt, or an ammonium salt, or a salt with an organic base such as methylamine, dimethylamine, trimethylamine, piperidine, morpholine or tris-(2-hydroxyethyl)amine.

Gemcitabine (Gemzar, trademark of Lilly Inc.) is the β-isomer of 2'-deoxy-2',2'-difluorocytidine monohydrochloride which has become a useful cytotoxic agent. It is a member of the antimetabolite class of cytotoxic agents.

As stated hereinbefore, the combination of the present invention comprising a Src inhibitor and gemcitabine is useful in the synergistic treatment or prophylaxis of cancer.

Cancers that are amenable to treatment with the combination of the present invention include oesophageal cancer, myeloma, hepatocellular, pancreatic and cervical cancer, Ewings tumour, neuroblastoma, kaposis sarcoma, ovarian cancer, breast cancer, colorectal cancer, prostate cancer, bladder cancer, melanoma, lung cancer [including non small cell lung cancer (NSCLC) and small cell lung cancer (SCLC)], gastric cancer, head and neck cancer, brain cancer, renal cancer, lymphoma and leukaemia. More particularly, the combination of the present invention is useful in the treatment or prevention of pancreatic cancer.

The cancer treatment of the present invention includes an anti-tumour effect that may be assessed by conventional means such as the response rate, the time to disease progression and/or the survival rate. Anti-tumour effects of the present invention include, but are not limited to, inhibition of tumour growth, tumour growth delay, regression of tumour, shrinkage of tumour, increased time to regrowth of tumour on cessation of treatment and slowing of disease progression. For example, it is expected that when the combination of the present invention is administered to a warm-blooded animal such as a human, in need of treatment for cancer involving a solid tumour, such a method of treatment will produce an effect, as measured by, for example, one or more of: the extent of the anti-tumour effect, the response rate, the time to disease progression and the survival rate.

As described hereinbefore, the combination of the present invention is useful in the synergistic treatment or prophylaxis of cancer. According to the present invention, a combination treatment is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination treatment is synergistic if the effect is therapeutically superior to the effect achievable with a Src inhibitor or gemcitabine alone. Further, the effect of the combination treatment is synergistic if a beneficial effect is obtained in a group of patients that does not respond (or responds poorly) to a Src inhibitor or gemcitabine alone. In addition, the effect of the combination treatment is defined as affording a synergistic effect if one of the components is dosed at its conventional dose and the other component is dosed at a reduced dose and the therapeutic effect, as measured by, for example, the extent of the response, the response rate, the time to disease progression or the survival period, is equivalent to that achievable on dosing conventional amounts of either one of the components of the combination treatment. In particular, synergy is deemed to be present if the conventional dose of the Src inhibitor or gemcitabine may be reduced without detriment to one or more of the extent of the response, the response rate, the time to disease progression and survival data, in particular without detriment to the duration of the response, but with fewer and/or less troublesome side-effects than those that occur when conventional doses of each component are used.

According to the present invention there is provided a combination comprising the Src inhibitor 4-(6-chloro-2,3-methylenedioxyanilino)-7-[2-(4-methylpiperazin-1-yl)ethoxy]-5-tetrahydropyran-4-yloxyquinazoline, or a pharmaceutically-acceptable acid-addition salt thereof, and gemcitabine for use in the synergistic treatment or prophylaxis of pancreatic cancer.

According to the present invention there is provided a combination comprising the Src inhibitor 4-(2-chloro-5-methoxyanilino)-6-methoxy-7-(N-methylpiperidin-4-ylmethoxy)quinazoline, or a pharmaceutically-acceptable acid-addition salt thereof, and gemcitabine for use in the synergistic treatment or prophylaxis of pancreatic cancer.

The therapeutic combination of the present invention may be administered in the form of a suitable pharmaceutical composition. According to this aspect of the invention there is provided a pharmaceutical composition for use in the synergistic treatment or prophylaxis of cancer which comprises a combination as defined hereinbefore in association with a pharmaceutically-acceptable excipient or carrier.

The compositions described herein may be in a form suitable for oral administration, for example as a tablet or capsule, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the Src inhibitor of the combination treatment may be delivered endoscopically, intratracheally, intralesionally, percutaneously, intravenously, subcutaneously, intraperitoneally or intratumourally. In general the compositions described herein may be prepared in a conventional manner using conventional excipients or carriers that are well known in the art.

Suitable phanmceutically-acceptable excipients or carriers for a tablet formulation include, for example, inert excipients such as lactose, sodium carbonate, calcium phosphate or calcium carbonate, granulating and disintegrating agents such as corn starch or alginic acid; binding agents such as gelatin or starch; lubricating agents such as magnesium stearate, stearic acid or talc; preservative agents such as ethyl or propyl 4-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case using conventional coating agents and procedures well known in the art.

Compositions for oral use may be in the form of hard gelatin capsules in which the active ingredient is mixed with an inert solid excipient, for example, calcium carbonate, calcium phosphate or kaolin, or as soft gelatin capsules in which the active ingredient is mixed with water or an oil such as peanut oil, liquid paraffin or olive oil.

The compositions of the present invention are advantageously presented in unit dosage form A Src inhibitor as defined hereinbefore will generally be administered so that a daily dose in the range, for example, 0.1 mg/kg to 75 mg/kg body weight is received, given if required in divided doses. In general lower doses will be administered when a parenteral route is employed. Thus, for example, for intravenous administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration is however preferred, particularly in tablet form. Typically, unit dosage forms will contain about 0.5 mg to 0.5 g of the Src inhibitor.

Gemcitabine may be administered according to known clinical practice. For example, in NSCLC the recommended dose of gemcitabine is 1000mg/m² given by 30 minute intravenous infusion. This may be repeated once weekly for three weeks, followed by a one week rest period. This four week cycle may then be repeated. Dosage reduction may be necessary if the patient experiences undue toxicity. In pancreatic cancer the recommended dose of gemcitabine is 1000mg/m² given by 30 minute intravenous infusion. This may be repeated once weekly for seven weeks followed by a week of rest. Subsequent cycles may consist of injections once weekly for three consecutive weeks out of every four weeks. Dosage reduction may be necessary if the patient experiences undue toxicity.

The dosages and schedules described hereinbefore may be varied according to the particular disease state and the overall condition of the patient. For example, it may be necessary or desirable to reduce the above-mentioned doses of the components of the combination treatment in order to reduce toxicity. Dosages and schedules may also vary if, in addition to a combination treatment of the present invention, one or more additional chemotherapeutic agents are used. Scheduling can be determined by the practitioner who is treating any particular patient using his professional skill and knowledge.

It will be appreciated that the pharmaceutical composition according to the present invention includes a composition comprising a Src inhibitor as defined hereinbefore and gemcitabine and a pharmaceutically-acceptable excipient or carrier. Such a composition conveniently provides the therapeutic combination product of the invention for simultaneous administration in the synergistic treatment or prophylaxis of cancer.

A pharmaceutical composition according to the present invention also includes separate compositions comprising a first composition comprising a Src inhibitor and a pharmaceutically-acceptable excipient or carrier, and a second composition comprising gemcitabine and a pharmaceutically-acceptable excipient or carrier. Such a composition conveniently provides the therapeutic combination of the invention for sequential or separate administration in the synergistic treatment or prophylaxis of cancer but the separate compositions may also be administered simultaneously.

Conveniently such a pharmaceutical composition of the invention comprises a kit comprising a first container with a suitable composition containing the Src inhibitor and a second container with a suitable composition containing gemcitabine. According to this aspect of the present invention there is provided a kit for use in the synergistic treatment or prophylaxis of cancer comprising :-
a) a Src inhibitor together with a pharmaceutically-acceptable excipient or carrier, in a first unit dosage form;
b) gemcitabine together with a pharmaceutically-acceptable excipient or carrier, in a second unit dosage form; and
c) container means for containing said first and second dosage forms.

According to this aspect of the invention there is also provided a pharmaceutical composition for use in the synergistic treatment or prophylaxis of pancreatic cancer which comprises a combination as defined hereinbefore in association with a pharmaceutically-acceptable excipient or carrier.

According to a further aspect of the present invention there is provided a combination as defined hereinbefore for use in the synergistic treatment or prophylaxis of cancer.

According to this aspect of the present invention there is also provided a combination as defined hereinbefore for use in the synergistic treatment or prophylaxis of pancreatic cancer.

According to a further aspect of the present invention there is provided the use of a combination as defined hereinbefore in the manufacture of a medicament for administration to a warm-blooded animal such as man to provide the synergistic treatment or prophylaxis of cancer.

According to this aspect of the present invention there is also provided the use of a combination as defined hereinbefore in the manufacture of a medicament for administration to a warm-blooded animal such as man to provide the synergistic treatment or prophylaxis of pancreatic cancer.

According to a further aspect of the present invention there is provided a method for the synergistic treatment or prophylaxis of cancer which comprises the administration to a warm-blooded animal such as man that is in need of such treatment of effective amounts of the components of the combination as defined hereinbefore.

According to this aspect of the present invention there is also provided a method for the synergistic treatment or prophylaxis of pancreatic cancer which comprises the administration to a warm-blooded animal such as man that is in need of such treatment of effective amounts of the components of the combination as defined hereinbefore.

According to this aspect of the present invention there is also provided a method for the synergistic treatment or prophylaxis of cancer which comprises the administration to a warm-blooded animal such as man that is in need of such treatment of an effective amount of a Src inhibitor as defined hereinbefore before, simultaneously with or after the administration of an effective amount of gemcitabine.

According to this aspect of the present invention there is also provided a method for the synergistic treatment or prophylaxis of cancer which comprises the simultaneous, sequential or separate administration to a warm-blooded animal such as man that is in need of such treatment of effective amounts of the components of the combination as defined hereinbefore.

According to this aspect of the present invention there is also provided a method for the synergistic treatment or prophylaxis of pancreatic cancer which comprises the simultaneous, sequential or separate administration to a warm-blooded animal such as man that is in need of such treatment of effective amounts of the components of the combination as defined hereinbefore.

According to this aspect of the present invention there is also provided a method for the synergistic treatment or prophylaxis of cancer which comprises the administration to a warm-blooded animal such as man that is in need of such treatment of an effective amount of a Src inhibitor as defined hereinbefore and the simultaneous, sequential or separate administration of an effective amount of gemcitabine.

According to this aspect of the present invention there is also provided a method for the synergistic treatment or prophylaxis of pancreatic cancer which comprises the administration to a warm-blooded animal such as man that is in need of such treatment of an effective amount of a Src inhibitor as defined hereinbefore and the simultaneous, sequential or separate administration of an effective amount of gemcitabine.

A combination treatment of the present invention as defined hereinbefore may be administered as a sole therapy or may in addition involve surgery or radiotherapy or the administration of an additional chemotherapeutic agent.

Surgery may comprise the step of partial or complete tumour resection, prior to, during or after the administration of the combination treatment of the present invention.

Other chemotherapeutic agents for optional use with the combination treatment of the present invention may include, for example, the following four categories of therapeutic agent :-
(i) antiproliferative/antineoplastic drugs and combinations thereof as used in medical oncology (for example carboplatin and cisplatin);
(ii) cytostatic agents;
(iii) biological response modifiers (for example interferon); and
(iv) antibodies (for example edrecolomab).

For example, the administration of a triple combination of a Src inhibitor as defined hereinbefore, gemcitabine and ionising radiation may produce anti-cancer effects, such as anti-tumour effects, that are greater than those achieved by the administration of any two components of the triple combination.

According to this aspect of the present invention there is provided a method for the synergistic treatment or prophylaxis of cancer which comprises the administration to a warm-blooded animal such as man that is in need of such treatment of an effective amount of a Src inhibitor as defined hereinbefore before, simultaneously with or after an effective amount of gemcitabine and before, simultaneously with or after an effective amount of ionising radiation.

According to this aspect of the present invention there is also provided a method for the synergistic treatment or prophylaxis of pancreatic cancer which comprises the administration to a warm-blooded animal such as man that is in need of such treatment of an effective amount of a Src inhibitor as defined hereinbefore before, simultaneously with or after an effective amount of gemcitabine and before, simultaneously with or after an effective amount of ionising radiation.

The ionising radiation may be given to said warm-blooded animal such as man within the period of a week before to a week after the administration of the combination of the present invention as defined hereinbefore.

Radiotherapy may be administered according to the known practices in clinical radiotherapy. The dosages of ionising radiation will be those known for use in clinical radiotherapy. The radiation therapy used will include for example the use of γ-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1Gy per hour over a number of days. Dosage ranges for radioisotopes vary widely, and depend on the half-life of the isotope, the strength and type of radiation emitted, and on the uptake by cells.

According to a further aspect of the present invention there is provided the use of a combination as defined hereinbefore in the manufacture of a medicament for administration to a warm-blooded animal such as man that is being treated with ionising radiation to provide the synergistic treatment or prophylaxis of cancer.

The following test method may be used to demonstrate the activity of the Src inhibitor 4-(2-chloro-5-methoxyanilino)-6-methoxy-7-(N-methylpiperidin-4-ylmethoxy)quinazoline (hereinafter identified by way of the code number Src 1) when administered in combination with gemcitabine.

The test method has been described by C J Bruns et al., Cancer Research, 2000, 60, 2926-2935 and involves the injection of pancreatic tumour cells derived from the COLO 357 human pancreatic cancer cell line into pancreas tissue in a group of nude mice and an evaluation of tumour growth and metastasis into liver node tissue.

L3.6p1 pancreatic cancer cells were obtained after successive cycles of cell selection from nude mouse tumour tissue that developed after injection of COLO 357 human pancreatic cancer cells. L3.6pl cancer cells (1x10⁶ cells) were injected into the pancreas of each animal in several groups of male athymic nude mice (n = 8 to 10 per group). After a period of 7 days, groups of test animals were treated with the test compound Src-1 (50 mg/kg or 25 mg/kg orally by gavage daily for 5 days per week on treatment days 1-5 and 8-12), with gemcitabine (100 mg/kg by intraperitoneal injection twice weekly on treatment days 2, 5, 9 and 12) or with a combination of both agents (*i.e*. gemcitabine by intraperitoneal injection twice weekly at 100 mg/kg on treatment days 2, 5, 9 and 12 and Src-1 at 50 mg/kg orally by gavage daily on treatment days 1-5 and 8-12).

On the days where both agents were given, the gemcitabine was dosed at least 1 hour before test compound Src-1. A control group of 10 mice received intraperitoneal injections of an equivalent volume of saline according to the same treatment schedule as the combination group. The animals were sacrificed 32 days after tumour cell injection. The pancreatic tumour weight was measured. The incidence of liver metastases was evaluated. All macroscopically enlarged liver nodules were evaluated by histopathology to confirm tumour metastasis.

The results are shown in the table which follows:-

| Treatment Group | Liver Metastases | Average Tumour Weight (mg) +/- std dev | Average Body Weight (g) +/- std dev |
|---|---|---|---|
| Control | 3/5 | 1359 +/- 397 | 24.2+/-1.9 |
| gemcitabine | 1/5 | 393 +/- 68 | 22.7 +/- 1.5 |
| Src-1 (50 mg/kg) | 0/9 | 827 +/- 176 | 22.3 +/- 6.8 |
| Src-1 (25 mg/kg) | 0/9 | 816 +/- 118 | 22.6 +/- 1.4 |
| Src-1 (50 mg/kg) + gemcitabine | 0/8 | 124 +/- 92 | 18.3 +/- 1.7 |

| | | | |
|---|---|---|---|
| Abbreviation std dev = standard deviation | | | |

The results demonstrate that, compared with the weight of control tumours, tumour growth in those animals treated with the combination of Src-1 (50 mg/kg) plus gemcitabine was much reduced (1359 mg and 124 mg respectively) to a level well below that achievable on the dosing of either gemcitabine of the Src inhibitor alone. In addition, there was no liver metastasis in the animals treated with the combination of Src-1 (50 mg/kg) plus gemcitabine whereas liver metastasis was present in 1/5 of the animals treated with gemcitabine alone.

### Src Inhibitors described within European Patent Applications 02292736.2 and 03290900.4

### Example 1

### 4-(5-chloro-2,3-methylenedioxypyrid-4-ylamino)-7-(3-chloropropoxy)-6-methoxyquinazoline

Sodium hexamethyldisilazane (1M solution in THF; 0.734 ml) was added to a solution of 4-amino-5-chloro-2,3-methylenedioxypyridine (0.12 g) in DMF (4 ml) that had been cooled to 0°C and the mixture was stirred for 15 minutes. A portion (0.1 g) of 4-chloro-7-(3-chloropropoxy)-6-methoxyquinazoline was added and the resultant mixture was stirred and allowed to warm to ambient temperature. The mixture was stirred at ambient temperature for 16 hours. The reaction mixture was evaporated and the residue was partitioned between methylene chloride and a saturated aqueous ammonium chloride solution. The organic phase was washed with water and with brine, dried over magnesium sulphate and evaporated. The residue was purified by column chromatography on silica using increasingly polar mixtures of methylene chloride and ethyl acetate as eluent followed by increasingly polar mixtures of methylene chloride and acetonitrile. There was thus obtained the title compound as a white foam (0.11 g); NMR Spectrum: (DMSOd₆ and CD₃CO₂D) 2.3 (m, 2H), 3.8 (m, 2H), 4.05 (s, 3H), 4.4 (t, 2H), 6.3 (s, 2H), 7.4 (s, 1H), 7.9 (s, 1H), 8.15 (s, 1H), 8.95 (s, 1H); Mass Spectrum: M+H⁺ 423 and 425.

The 4-amino-5-chloro-2,3-methylenedioxypyridine used as a starting material was prepared as follows:-
Bromochloromethane (20 ml) was added to a mixture 5-chloro-2,3-dihydroxypyridine (30 g), caesium carbonate (100 g) and DMF (300 ml) and the mixture was stirred and heated to 90°C for 3.5 hours. The mixture was cooled to ambient temperature and filtered. The filtrate was evaporated and the residue was purified by column chromatography on silica using methylene chloride as eluent. There was thus obtained 5-chloro-2,3-methylenedioxypyridine as a white solid (4.7 g); NMR Spectrum: (DMSOd₆) 6.25 (s, 2H), 7.5 (s, 1H), 7.65 (s, 1H).
A mixture of diisopropylamine (8.2 ml) and THF (100 ml) was cooled to -70°C and n-butyllithium (2.5 M in hexane, 24 ml) was added dropwise. The mixture was stirred at -70°C for a further 20 minutes. A solution of 5-chloro-2,3-methylenedioxypyridine (4.2 g) in THF (40 ml) was added over 10 minutes and the reaction mixture was stirred at -70°C for 1 hour. Dry carbon dioxide gas was bubbled into the reaction mixture for 30 minutes. The resultant reaction mixture was allowed to warm to ambient temperature. Water (20 ml) was added and the organic solvent was evaporated. The residue was acidified to pH2 by the addition of 1N aqueous hydrochloric acid solution. The resultant solid was isolated and washed in turn with water and diethyl ether and dried under vacuum at 40°C. There was thus obtained 5-chloro-2,3-methylenedioxypyridine-4-carboxylic acid (3.6 g); ¹³C NMR Spectrum: (DMSOd₆) 103, 120, 121, 138, 140, 158, 163.

A mixture of the material so obtained, diphenylphosphoryl azide (3.6 ml), anhydrous tert-butanol (13.5 ml), triethylamine (4.2 ml) and 1,4-dioxane (63 ml) was stirred and heated to 100°C for 3 hours. The mixture was evaporated and the residue was partitioned between ethyl acetate and water. The organic phase was washed with water, dried over magnesium sulphate and evaporated. The residue was purified by column chromatography on silica using a 9:1 mixture of methylene chloride and ethyl acetate as eluent. There was thus obtained tert-butyl 5-chloro-2,3-methylenedioxypyrid-4-ylcarbamate (3.8 g); NMR Spectrum: (DMSOd₆) 1.45 (s, 9H), 6.2 (s, 2H), 7.7 (s, 1H), 9.2 (s, 1H).

The material so obtained was dissolved in methylene chloride (35 ml) and the solution was cooled to 0°C. Trifluoroacetic acid (15 ml) was added and the mixture was stirred at 0°C for 3 hours. The mixture was allowed to warm to ambient temperature and was stirred for 16 hours. The solvent was evaporated and the residue was diluted with ice water and neutralised to pH7 by the addition of 2N aqueous sodium hydroxide solution whilst keeping the mixture temperature at 0°C. The resultant mixture was extracted with methylene chloride and the extract dried over magnesium sulphate and evaporated. The residue was purified by column chromatography on silica using a 19:1 mixture of methylene chloride and diethyl ether as eluent. There was thus obtained 4-amino-5-chloro-2,3-methylenedioxypyridine (2 g); NMR Spectrum: (DMSOd₆) 6.1 (s, 2H), 6.2 (s, 2H), 7.45 (s, 1H); ¹³C NMR Spectrum: (DMSOd₆) 100, 112, 125, 136, 138, 157; Mass Spectrum: M+H⁺ 173.

The 4-chloro-7-(3-chloropropoxy)-6-methoxyquinazoline used as a starting material was prepared as follows:-
Ammonium formate (45 g) was added portionwise over 1.25 hours to a stirred mixture of 7-benzyloxy-6-methoxy-3,4-dihydroquinazolin-4-one (International Patent Application WO 02/16352, Example 1 thereof; 20 g), 10% palladium-on-carbon catalyst (3.3 g) and DMF (530 ml) and the reaction mixture was stirred for an additional 30 minutes. The catalyst was removed by filtration and the solvent was evaporated. There was thus obtained 7-hydroxy-6-methoxy-3,4-dihydroquinazolin-4-one (8.65 g); NMR Spectrum: (DMSOd₆) 3.9 (s, 3H), 7.0 (s, 1H), 7.45 (s, 1H), 7.9 (s, 1H).

A mixture of the material so obtained, acetic anhydride (63 ml) and pyridine (7.5 ml) was heated to 100°C for 4.5 hours. The resultant mixture was allowed to stand at ambient temperature for 16 hours. The mixture was poured into a stirred mixture (400 ml) of ice and water. The resultant precipitate was isolated and dried under vacuum. Analysis revealed that hydrolysis of the acetate group on the 4-position of the quinazoline was incomplete. The mixture was therefore further hydrolysed with water (150 ml) and pyridine (a few drops) at 90°C for 15 minutes. The resultant mixture was cooled to ambient temperature and the solid was collected by filtration, washed with water and dried under vacuum There was thus obtained 7-acetoxy-6-methoxy-3,4-dihydroquinazolin-4-one (7.4 g); NMR Spectrum: (DMSOd₆) 2.3 (s, 3H), 3.9 (s, 3H), 7.45 (s, 1H), 7.65 (s, 1H), 8.05 (s, 1H).

A mixture of a portion (2 g) of the material so obtained, thionyl chloride (32 ml) and DMF (5 drops) was stirred and heated to reflux for 1.5 hours. The mixture was cooled to ambient temperature and the excess of thionyl chloride was evaporated. Toluene was added to the residue and evaporated. The resultant residue was diluted with methylene chloride (15 ml) and a 10:1 mixture (80 ml) of methanol and a saturated aqueous ammonium hydroxide solution was added. The resultant mixture was stirred and heated to 80°C for 10 minutes. The mixture was cooled to ambient temperature and evaporated. Water was added to the residue and the mixture was neutralised by the addition of dilute aqueous hydrochloric acid solution. The resultant precipitate was collected by filtration and dried under vacuum at 35°C for 16 hours. There was thus obtained 4-chloro-7-hydroxy-6-methoxyquinazoline (1.65 g); NMR Spectrum: (DMSOd₆) 4.0 (s, 3H), 7.25 (s, 1H), 7.4 (s, 1H), 8.8 (s, 1H).

Di-tert-butyl azodicarboxylate (2.3 g) was added portionwise over a few minutes to a stirred mixture of 4-chloro-7-hydroxy-6-methoxyquinazoline (1.65 g), 3-chloropropanol (0.7 ml), triphenylphosphine (2.6 g) and methylene chloride (100 ml) and the reaction mixture was stirred at ambient temperature for 2 hours. The mixture was concentrated to a volume of about 30 ml by evaporation and the residue was purified by column chromatography on silica using increasingly polar mixtures of petroleum ether (b.p 40-60°C) and ethyl acetate as eluent. There was thus obtained 4-chloro-7-(3-chloropropoxy)-6-methoxyquinazoline as a white solid (2 g); NMR Spectrum: (DMSOd₆) 2.3 (m, 2H), 3.8 (m, 2H), 4.05 (s, 3H), 4.4 (m, 2H), 7.45 (s, 1H), 7.55 (s, 1H), 8.9 (s, 1H).

### Example 2

### 7-(2-chloroethoxy)-4-(5-chloro-2,3-methylenedioxypyrid-4-ylamino)-6-methoxyquinazoline

Using an analogous procedure to that described in Example 1, 4-chloro-7-(2-chloroethoxy)-6-methoxyquinazoline was reacted with 4-amino-5-chloro-2,3-methylenedioxypyridine to give the title compound in 92% yield; NMR Spectrum: (DMSOd₆ and CD₃CO₂D) 4.05 (s, 3H), 4.1 (t, 2H), 4.55 (t, 2H), 6.3 (s, 2H), 7.4 (s, 1H), 7.9 (s, 1H), 8.15 (s, 1H), 8.95 (s, 1H); Mass Spectrum: M+H⁺ 409 and 411.

The 4-chloro-7-(2-chloroethoxy)-6-methoxyquinazoline used as a starting material was prepared as follows:-
1,2-Dichloroethane (400 ml) was added to a stirred mixture of 7-hydroxy-6-methoxy-3-pivaloyloxymethyl-3,4-dihydroquinazolin-4-one (International Patent Application WO 02/16352, Example 2, Note [4] thereof; 85 g), potassium carbonate (77 g) and DMF (400 ml) and the reaction mixture was heated to 70°C for 16 hours. The reaction mixture was cooled to ambient temperature and filtered. The filtrate was evaporated and the solid so obtained was washed with water and dried over phosphorus pentoxide at 50°C. The material so obtained was purified by column chromatography on silica using increasingly polar mixtures of methylene chloride and ethyl acetate as eluent. There was thus obtained 7-(2-chloroethoxy)-6-methoxy-3-pivaloyloxymethyl-3,4-dihydroquinazolin-4-one as a white solid (65.6 g); NMR Spectrum: (CDCl₃) 1.2 (s, 9H), 3.9 (t, 2H), 4.0 (s, 3H), 4.4 (t, 2H), 5.95 (s, 2H), 7.1 (s, 1H), 7.7 (s, 1H), 8.2 (s, 1H); Mass Spectrum: M+H⁺ 369 and 371.

A mixture of the material so obtained and a saturated solution of ammonia gas in methanol (1.6 L) was stirred at ambient temperature for 2 days. The solvent was concentrated by evaporation to about one-fourth of the original volume and the precipitate was collected by filtration and washed with diethyl ether. There was thus obtained 7-(2-chloroethoxy)-6-methoxy-3,4-dihydroquinazolin-4-one as a white solid (44 g); NMR Spectrum: (DMSOd₆) 3.9 (s, 3H), 4.05 (t, 2H), 4.4 (t, 2H), 7.15 (s, 1H), 7.45 (s, 1H), 8.0 (s, 1H); Mass Spectrum: M+H⁺ 255 and 257.

A mixture of a portion (5 g) of the material so obtained, thionyl chloride (28 ml) and DMF (0.7 ml) was stirred and heated to 80°C for 1.5 hours. The excess of thionyl chloride was evaporated and toluene was added and evaporated. The residual solid was suspended in a mixture of ice and water and basified to pH7.5 by the addition of 2N aqueous sodium hydroxide solution followed by a saturated aqueous sodium bicarbonate solution. The resultant solid was collected by filtration, washed with water and diethyl ether and dried over over phosphorus pentoxide under vacuum. The material so obtained was purified by column chromatography on silica using increasingly polar mixtures of methylene chloride and acetonitrile as eluent. There was thus obtained 4-chloro-7-(2-chloroethoxy)-6-methoxyquinazoline (3.06 g); NMR Spectrum: (CDCl₃) 3.95 (t, 2H), 4.1 (s, 3H), 4.5 (t, 2H), 7.35 (s, 1H), 7.45 (s, 1H), 8.9 (s, 1H); Mass Spectrum: M+H⁺ 273 and 275.

### Example 3

### 4-(5-chloro-2,3-methylenedioxypyrid-4-ylamino)-6-methoxy-7-[3-(4-prop-2-ynylpiperazin-1-yl)propoxy]quinazoline

A mixture of 4-(5-chloro-2,3-methylenedioxypyrid-4-ylamino)-7-(3-chloropropoxy)-6-methoxyquinazoline (0.08 g), 1-prop-2-ynylpiperazine (0.047 g), potassium iodide (0.01 g) and DMA (2 ml) was stirred and heated to 80°C for 3.5 hours. The solvent was evaporated and the residue was partitioned between methylene chloride and a saturated aqueous ammonium chloride solution. The organic phase was dried over magnesium sulphate and evaporated. The residue was purified by column chromatography on silica using a 19:1 mixture of methylene chloride and methanol and then a 9:1 mixture of methylene chloride and a saturated methanolic ammonia solution as eluent. The resulting gum was triturated under diethyl ether. There was thus obtained the title compound as a solid (0.066 g); NMR Spectrum: (DMSOd₆ and CF₃CO₂D) 2.3 (m, 2H), 3.2-3.6 (br m, 10H), 3.75 (s, 1H), 3.95 (br s, 2H), 4.0 (s, 3H), 4.35 (m, 2H), 6.3 (s, 2H), 7.4 (s, 1H), 7.9 (s, 1H), 8.15 (s, 1H), 8.95 (s, 1H); Mass Spectrum: M+H⁺ 511 and 513.

The 1-prop-2-ynylpiperazine used as a starting material was prepared as follows :-
Propargyl bromide (80% solution in toluene; 40 ml) was added dropwise during 10 minutes to a stirred mixture of 1-tert-butoxycarbonylpiperazine (50 g), potassium carbonate (74.2 g) and acetonitrile (2 L) that had been cooled to 0°C. The mixture was stirred for 1.5 hours and allowed to warm to ambient temperature. The mixture was filtered and the filtrate was evaporated. The residue was purified by column chromatography on silica using increasingly polar mixtures of methylene chloride and ethyl acetate as eluent. There was thus obtained tert-butyl 4-prop-2-ynylpiperazine-1-carboxylate as an oil (45.5 g); NMR Spectrum: (CDCl₃) 1.4 (s, 9H), 2.2 (s, 1H), 2.45 (m, 4H), 3.3 (s, 2H), 3.45 (m, 4H).

A solution of the material so obtained in methylene chloride (100 ml) was added slowly to a solution of hydrogen chloride gas in 1,4-dioxane (4M, 450 ml). The reaction was slightly exothermic and a precipitate formed as carbon dioxide gas was evolved. The mixture was stirred at ambient temperature for 1 hour. The resultant mixture was evaporated and the residue was suspended in methylene chloride. A solution of ammonia gas in methanol (7M, 110 ml) was added and the mixture was stirred at ambient temperature for 15 minutes. The mixture was filtered and the filtrate was evaporated. An oil was obtained which crystallised on standing. There was thus obtained 1-prop-2-ynylpiperazine (23 g); NMR Spectrum: (CDCl₃) 2.2 (s, 1H), 2.5 (br s, 4H), 2.85 (m, 4H), 3.25 (s, 2H).

### Example 4

### 7-(2-chloroethoxy)-4-(5-chloro-2,3-methylenedioxypyrid-4-ylamino)-5-tetrahydropyran-4-yloxyquinazoline

Using an analogous procedure to that described in Example 1, 4-chloro-7-(2-chloroethoxy)-5-tetrahydropyran-4-yloxyquinazoline was reacted with 4-amino-5-chloro-2,3-methylenedioxypyridine to give the title compound in 37% yield; NMR Spectrum: (CDCl₃) 2.0 (m, 2H), 2.3 (m, 2H), 3.65 (m, 2H), 3.9 (m, 2H), 4.1 (m, 2H), 4.4 (m, 2H), 4.8 (m, 6.2 (s, 2H), 6.65 (s, 1H), 6.9 (s, 1H), 7.8 (s, 1H), 8.6 (s, 1H), 9.5 (s, 1H); Mass Spectrum: M+H⁺ 479 and 481.

The 4-chloro-7-(2-chloroethoxy)-5-tetrahydropyran-4-yloxyquinazoline used as a starting material was prepared as follows:-
Di-tert-butyl azodicarboxylate (0.338 g) was added to a stirred mixture of 4-chloro-7-hydroxy-5-tetrahydropyran-4-yloxyquinazoline (International Patent Application WO 01/94341, Example 15, Note [10] thereof; 0.25 g), 2-chloroethanol (0.073 ml), triphenylphosphine (0.385 g) and methylene chloride (15 ml) and the reaction mixture was stirred at ambient temperature for 1 hour. The mixture was concentrated to a volume of about 5 ml by evaporation and the residue was purified by column chromatography on silica using increasingly polar mixtures of petroleum ether (b.p 40-60°C) and ethyl acetate as eluent. There was thus obtained 4-chloro-7-(2-chloroethoxy)-5-tetrahydropyran-4-yloxyquinazoline as a solid (0.17 g); NMR Spectrum: (CDCl₃) 2.0 (m, 2H), 2.15 (m, 2H), 3.7 (m, 2H), 3.95 (t, 2H), 4.1 (m, 2H), 4.4 (t, 2H), 4.8 (m, 1H), 6.7 (s, 1H), 6.95 (s, 1H), 8.85 (s, 1H).

### Example 5

### 7-(2-chloroethoxy)-4-(5-chloro-2,3-methylenedioxypyrid-4-ylamino)-5-isopropoxyquinazoline

Using an analogous procedure to that described in Example 1, 4-chloro-7-(2-chloroethoxy)-5-isopropoxyquinazoline was reacted with 4-amino-5-chloro-2,3-methylenedioxypyridine to give the title compound in 86% yield; NMR Spectrum: (CDCl₃) 1.55 (d, 6H), 3.9 (t, 2H), 4.4 (t, 2H), 4.9 (m, 1H), 6.2 (s, 2H), 6.6 (s, 1H), 6.85 (s, 1H), 7.75 (s, 1H), 8.6 (s, 1H), 9.65 (s, 1H); Mass Spectrum: M+H⁺ 437 and 439.

The 4-chloro-7-(2-chloroethoxy)-5-isopropoxyquinazoline used as a starting material was prepared as follows:-
Di-tert-butyl azodicarboxylate (28.9 g) was added to a stirred mixture of 7-benzyloxy-5-hydroxy-3-pivaloyloxymethyl-3,4-dihydroquinazolin-4-one (International Patent Application WO 01/94341, Example 15, Note [8] thereof; 30 g), isopropanol (7.3 ml), triphenylphosphine (32.95 g) and methylene chloride (350 ml) that had been cooled to 0°C. The reaction mixture was allowed to warm to ambient temperature and was stirred for 1.5 hours. The mixture was evaporated and the residue was purified by column chromatography on silica using increasingly polar mixtures of methylene chloride and methanol as eluent. There was thus obtained 7-benzyloxy-5-isopropoxy-3,4-dihydroquinazolin-4-one as a solid (23.8 g); NMR Spectrum: (DMSOd₆) 7.89 (s, 1H), 7.5-7.3 (m, 5H), 6.75 (s, 1H), 6.62 (s, 1H), 5.24 (s, 2H), 4.65 (m, 1H), 1.29 (d, 6H).

Ammonium formate (48.4 g) was added to a stirred mixture of 7-benzyloxy-5-isopropoxy-3,4-dihydroquinazolin-4-one (23.8 g), 10% palladium-on-carbon catalyst (2.8 g) and DMF (300 ml) and the resultant mixture was stirred at ambient temperature for 2 hours. The mixture was filtered and the filtrate was evaporated. The material so obtained was triturated under water, the pH of which was adjusted to pH7. The solid so obtained was collected by filtration, washed with water and with diethyl ether and dried over phosphorus pentoxide under vacuum There was thus obtained 7-hydroxy-5-isopropoxy-3,4-dihydroquinazolin-4-one as a white solid (15.9 g); NMR Spectrum: (DMSOd₆) 1.3 (d, 6H), 4.57 (m, 1H), 6.42 (s, 1H), 6.5 (s, 1H), 7.8 (s, 1H).

A mixture of the material so obtained, acetic anhydride (34 ml) and pyridine (0.62 ml) was heated to 70°C for 30 minutes. The reaction mixture was cooled to ambient temperature and the excess of acetic anhydride was evaporated. The white solid so obtained was added to hot water (80°C, 250 ml) and the mixture was stirred vigorously and heated to 80°C for 20 minutes. The mixture was cooled to ambient temperature and the solid was isolated and dried over phosphorus pentoxide. There was thus obtained 7-acetoxy-5-isopropoxy-3,4-dihydroquinazolin-4-one (17.86 g); NMR Spectrum: (DMSOd₆) 7.97 (s, 1H), 6.91 (s, 1H), 6.85 (s, 1H), 4.65 (m, 1H), 2.32 (s, 3H), 1.33 (d, 6H).

A mixture of a portion (5.4 g) of the material so obtained, triphenylphosphine (10.8 g), carbon tetrachloride (12 ml) and 1,2-dichloroethane (50 ml) was stirred and heated to 70°C for 2 hours. The mixture was cooled to ambient temperature and the solvent was evaporated. The residue was dissolved in a 0.5M solution of ammonia gas in 1,4-dioxane (250 ml) and the mixture was heated to 70°C for 10 minutes. The solvent was evaporated and the residue was cooled in an ice-water bath. Methylene chloride and water were added and the aqueous layer was brought to pH7 by the addition of dilute aqueous hydrochloric acid. The mixture was filtered. The organic phase was dried over magnesium sulphate and evaporated to give 4-chloro-7-hydroxy-5-isopropoxyquinazoline as a foam which was used without further purification.

Di-tert-butyl azodicarboxylate (7.9 g) was added to a stirred mixture of the 4-chloro-7-hydroxy-5-isopropoxyquiuazoline so obtained, 2-chloroethanol (1.5 ml), triphenylphosphine (8 g) and methylene chloride (200 ml) and the reaction mixture was stirred at ambient temperature for 4 hours. The mixture was concentrated by evaporation and the residue was purified by column chromatography on silica using increasingly polar mixtures of petroleum ether (b.p 40-60°C) and ethyl acetate as eluent. There was thus obtained 4-chloro-7-(2-chloroethoxy)-5-isopropoxyquinazoline (2.5 g); NMR Spectrum: (CDCl₃) 1.45 (d, 6H), 3.9 (t, 2H), 4.4 (t, 2H), 4.75 (m, 1H), 6.65 (s, 1H), 6.9 (s, 1H), 8.8 (s, 1H).

### Example 6

Using an analogous procedure to that described in Example 3, the appropriate 7-haloalkoxyquinazoline was reacted with the appropriate heterocyclic compound to give the compounds described in Table I. Unless otherwise stated, each compound described in Table I was obtained as a free base.

**Table I**

| | | |
|---|---|---|
| 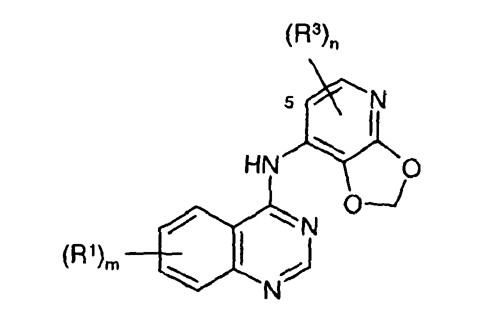 | | |

| Compound No. & Note | (R¹)ₘ | (R³)ₙ |
|---|---|---|
| [1] | 6-methoxy-7-[3-(4-isobutyrylpiperazin-1-yl)propoxy] | 5-chloro |
| [2] | 6-methoxy-7- {3-[4-(2,2,2-trifluoroethyl)piperazin-1-yl]propoxy} | 5-chloro |
| [3] | 6-methoxy-7-[2-(4-prop-2-ynylpiperazin-1-yl)ethoxy] | 5-chloro |
| [4] | 5-tetrahydropyran-4-yloxy-7-[2-(4-acetylpiperazin-1-yl)ethoxy] | 5-chloro |
| [5] | 5-tetrahydropyran-4-yloxy-7-{2-[(3RS,4SR)-3,4-methylenedioxypyrrolidin-1-yl]ethoxy} | 5-chloro |
| [6] | 5-isopropoxy-7-[2-(4-acetylpiperazin-1-yl)ethoxy] | 5-chloro |
| [7] | 5-isopropoxy-7-{2-[(3RS,4SR)-3,4-methylenedioxypyrrolidin-1-yl]ethoxy} | 5-chloro |
| [8] | 6-(2-morpholinoethoxy)-7-methoxy | 5-chloro |
| [9] | 6-[2-(4-methylpiperazin-1-yl)ethoxy]-7-methoxy | 5-chloro |
| [10] | 6-(2-pyrrolidin-1-ylethoxy)-7-methoxy | 5-chloro |
| [11] | 6-[2-(4-acetylpiperazin-1-yl)ethoxy]-7-methoxy | 5-chloro |
| [12] | 6-{2-[(3RS,4SR)-3,4-methylenedioxypyrrolidin-1-yl]ethoxy}-7-methoxy | 5-chloro |
| [13] | 6-(3-pyrrolidin-1-ylpropoxy)-7-methoxy | 5-chloro |
| [14] | 6-(3-morpholinopropoxy)-7-methoxy | 5-chloro |
| [15] | 6-[3-(4-acetylpiperazin-1-yl)propoxy]-7-methoxy | 5-chloro |
| [16] | 6-[3-(4-methylpiperazin-1-yl)propoxy]-7-methoxy | 5-chloro |
| [17] | 6-{3-[(3RS,4SR)-3,4-methylenedioxypyrrolidin-1-yl]propoxy}-7-methoxy | 5-chloro |
| [18] | 5-tetrahydropyran-4-yloxy-7-[2-(4-prop-2-ynylpiperazin-1-yl)ethoxy] | 5-chloro |
| [19] | 5-tetrahydropyran-4-yloxy-7-(2-morpholinoethoxy) | 5-chloro |
| [20] | 5-tetrahydropyran-4-yloxy-7-(3-morpholinopropoxy) | 5-chloro |
| [21] | 5-tetrahydropyran-4-yloxy-7-[3-(4-prop-2-ynylpiperazin-1-yl)propoxy] | 5-chloro |
| [22] | 5-isopropoxy-7-(2-piperazin-1-ylethoxy) | 5-chloro |
| [23] | 5-isopropoxy-7-{2-[4-(2-hydroxyethyl)piperazin-1-yl] ethoxy} | 5-chloro |
| [24] | 5-isopropoxy-7-(2-pyrrolidin-1-ylethoxy) | 5-chloro |
| [25] | 5-isopropoxy-7-(2-piperidinoethoxy) | 5-chloro |
| [26] | 5-isopropoxy-7-(2-morpholinoethoxy) | 5-chloro |
| [27] | 5-isopropoxy-7-[2-(4-prop-2-ynylpiperazin-1-yl)ethoxy] | 5-chloro |
| [28] | 5-isopropoxy-6-{2-[(3RS,4SR)-3,4-dimethoxypyrrolidin-1-yl]ethoxy} | 5-chloro |
| [29] | 6-{2-[(3RS,4SR)-3,4-ethylidenedioxypyrrolidin-1-yl]ethoxy}-5-isopropoxy | 5-chloro |
| [30] | 5-isopropoxy-7-[2-(4-methylpiperazin-1-yl)ethoxy] | 5-chloro |
| [31] | 5-isopropoxy-7-(3-morpholinopropoxy) | 5-chloro |
| [32] | 7-(3-morpholinopropoxy) | 5-chloro |
| [33] | 7-[3-(4-acetylpiperazin-1-yl)propoxy] | 5-chloro |
| [34] | 6-methoxy-7-[2-(4-prop-2-ynylpiperazin-1-yl)ethoxy] | hydrogen |
| [35] | 6-methoxy-7-[3-(4-prop-2-ynylpiperazin-1-yl)propoxy] | hydrogen |

### Notes

[1] The reactants were 4-(5-chloro-2,3-methylenedioxypyrid-4-ylamino)-7-(3-chloropropoxy)-6-methoxyquinazoline and 1-isobutyrylpiperazine. The reaction mixture was heated to 120°C for 3 hours. The reaction product was purified by column chromatography on a C18 reversed phase silica column (Waters Symmetry column, 5 microns silica, 19 mm diameter, 100 mm length) using a decreasingly polar mixture of water and acetonitrile (containing 1% acetic acid) as eluent. The material so obtained was dissolved in methylene chloride and an ion exchange resin (diethylaminopolystyrene resin, 4 equivalents) was added and the mixture was stirred for 30 minutes. The mixture was filtered and the filtrate was evaporated. The resultant residue was triturated under pentane to give the required product in 51 % yield which gave the following characterising data; NMR Spectrum: (CDCl₃) 1.1 (d, 6H), 2.1 (m, 2H), 2.45 (m, 4H), 2.55 (m, 2H), 2.75 (m, 1H), 3.5 (m, 2H), 3.6 (m, 2H), 4.0 (s, 3H), 4.25 (t, 2H), 6.1 (s, 2H), 7.1 (br s, 1H), 7.3 (s, 1H), 7.75 (s, 1H), 8.7 (br s, 1H); Mass Spectrum: M+H⁺ 543 and 545.
   The 1-isobutyrylpiperazine used as a starting material was prepared as follows :-
   Isobutyryl chloride (3.25 ml) was added dropwise to a stirred mixture of 1-benzylpiperazine (5 g), triethylamine (4.35 ml) and methylene chloride (75 ml) which was cooled to 0°C. The reaction mixture was allowed to warm to ambient temperature and stirred for 1 hour. The mixture was partitioned between methylene chloride and water. The organic phase was washed with water and with brine, dried over magnesium sulphate and evaporated. The residue was purified by column chromatography on silica using a 3:2 mixture of methylene chloride and ethyl acetate as eluent. There was thus obtained 1-benzyl-4-isobutyrylpiperazine (5.95 g) as an oil; NMR Spectrum: (CDCl₃) 1.1 (d, 6H), 2.45 (m, 4H), 2.8 (m, 1H), 3.5 (m, 4H), 3.65 (m, 2H), 7.3 (m, 5H); Mass Spectrum: M+H⁺ 247.
      A mixture of the material so obtained, cyclohexene (70 ml), palladium oxide-on-carbon catalyst (20%; 1.1 g) and ethanol (120 ml) was stirred and heated to 80°C for 3 hours. The catalyst was removed by filtration and the solvent was evaporated to give 1-isobutyrylpiperazine (3.7 g) as a solid; NMR Spectrum: (CDCl₃) 1.05 (d, 6H), 2.75 (m, 1H), 2.8 (m, 4H), 3.45 (m, 2H), 3.55 (m, 2H).
[2] The reactants were 4-(5-chloro-2,3-methylenedioxypyrid-4-ylamino)-7-(3-chloropropoxy)-6-methoxyquinazoline and 1-(2,2,2-trifluoroethyl)piperazine. The reaction mixture was heated to 120°C for 3 hours. The reaction product was purified by column chromatography on a C18 reversed phase silica column (Waters Symmetry column, 5 microns silica, 19 mm diameter, 100 mm length) using a decreasingly polar mixture of water and acetonitrile (containing 1% acetic acid) as eluent. The material so obtained was dissolved in methylene chloride and an ion exchange resin (diethylaminopolystyrene resin, 4 equivalents) was added and the mixture was stirred for 30 minutes. The mixture was filtered and the filtrate was evaporated. The resultant residue was triturated under pentane to give the required product in 72% yield which gave the following characterising data; NMR Spectrum: (CDCl₃) 2.1 (m, 2H), 2.5 (m, 6H), 2.7 (m, 4H), 2.95 (q, 2H), 4.05 (s, 3H), 4.25 (t, 2H), 6.1 (s, 2H), 7.1 (br s, 1H), 7.3 (s, 1H), 7.75 (s, 1H), 8.35 (br s, 1H); Mass Spectrum: M+H⁺ 555 and 557; Elemental Analysis: Found C, 51.8; H, 5.0; N, 14.8; C₂₄H₂₆ClF₃N₆O₄ requires C, 51.9; H, 4.7; N, 15.1%.
   The 1-(2,2,2-trifluoroethyl)piperazine used as a starting material was prepared as follows:-
   2,2,2-Trifluoroethyl trifluoromethanesulphonate (8.2 g) was added to a stirred mixture of 1-tert-butoxycarbonylpiperazine (6 g), potassium carbonate (5.77 g) and acetonitrile (30 ml) and the resultant mixture was stirred at ambient temperature for 16 hours. The mixture was filtered and the filtrate was evaporated. The residue was purified by column chromatography on silica using increasingly polar mixtures of petroleum ether (b.p 40-60°C) and ethyl acetate as eluent. There was thus obtained tert-butyl 4-(2,2,2-trifluoroethylpiperazine-1-carboxylate as a solid (8.1 g); NMR Spectrum: (CDCl₃) 1.45 (s, 9H), 2.6 (m, 4H), 2.95 (q, 2H), 3.4 (m, 4H).
      Hydrogen chloride gas was bubbled through a solution of tert-butyl 4-(2,2,2-trifluoroethylpiperazine-1-carboxylate (8 g) in ethyl acetate (50 ml) during 1.5 hours. A precipitate formed as carbon dioxide gas was evolved. The precipitate was collected by filtration, washed with ethyl acetate and dried under vacuum There was thus obtained 1-(2,2,2-trifluoroethyl)piperazine hydrochloride (7 g); NMR Spectrum: (DMSOd₆ and CF₃CO₂D) 2.85 (m, 4H), 3.1 (m, 4H), 3.35 (q, 2H).
      The material so obtained was suspended in methylene chloride and a saturated methanolic ammonia solution (20 ml) was added. The resultant mixture was stirred at ambient temperature for 20 minutes. The mixture was filtered and the filtrate was evaporated at ambient temperature under vacuum. There was thus obtained 1-(2,2,2-trifluoroethyl)piperazine which was used without any additional purification.
[3] The reactants were 7-(2-chloroethoxy)-4-(5-chloro-2,3-methylenedioxypyrid-4-ylamino)-6-methoxyquinazoline and 1-prop-2-ynylpiperazine. The required product was obtained in 52% yield and gave the following characterising data; NMR Spectrum: (DMSOd₆ and CF₃CO₂D) 3.3 (br s, 4H), 3.6 (br s, 4H), 3.75 (br s, 3H), 3.95 (s, 2H), 4.05 (s, 3H), 4.65 (t, 2H), 6.3 (s, 2H), 7.5 (s, 1H), 7.9 (s, 1H), 8.2 (s, 1H), 9.0 (s, 1H); Mass Spectrum: M+H⁺ 497 and 499; Elemental Analysis: Found C, 56.3; H, 5.4; N, 16.2; C₂₄H₂₅ClN₆O₄ 0.7H₂O requires C, 56.6; H, 5.2; N, 16.5%.
[4] The reactants were 7-(2-chloroethoxy)-4-(5-chloro-2,3-methylenedioxypyrid-4-ylamino)-5-tetrahydropyran-4-yloxyquinazoline and 1-acetylpiperazine. The reaction mixture was heated to 80°C for 3 hours and then to 110°C for 5 hours. The reaction product was purified by column chromatography on a C 18 reversed phase silica column (Waters Symmetry column, 5 microns silica, 19 mm diameter, 100 mm length) using a decreasingly polar mixture of water and acetonitrile (containing 1% acetic acid) as eluent. The organic solvents were evaporated and the pH of the aqueous phase was adjusted to 7.5. The solution was extracted with methylene chloride and the organic phase was dried over magnesium sulphate and evaporated. The resultant residue was triturated under diethyl ether to give the required product in 45% yield which gave the following characterising data; NMR Spectrum: (CDCl₃) 2.0 (m, 2H), 2.1 (s, 3H), 2.3 (m, 2H), 2.6 (m, 4H), 2.95 (m, 2H), 3.55 (m, 2H), 3.65 (m, 4H), 4.1 (m, 2H), 4.3 (m, 2H), 4.8 (m, 1H), 6.2 (s, 2H), 6.6 (s, 1H), 6.9 (s, 1H), 7.8 (s, 1H), 8.65 (s, 1H), 9.5 (s, 1H); Mass Spectrum: M+H⁺ 571 and 573; Elemental Analysis: Found C, 55.3; H, 5.4; N, 13.9; C₂₇H₃₁ClN₆O₆ 1H₂O requires C, 55.1; H, 5.7; N, 14.3.
[5] The reactants were 7-(2-chloroethoxy)-4-(5-chloro-2,3-methylenedioxypyrid-4-ylamino)-5-tetrahydropyran-4-yloxyquinazoline and (3RS,4SR)-3,4-methylenedioxypyrrolidine. The reaction mixture was heated to 80°C for 3 hours and then to 110°C for 5 hours. The reaction product was purified by column chromatography on a C 18 reversed phase silica column (Waters Symmetry column, 5 microns silica, 19 mm diameter, 100 mm length) using a decreasingly polar mixture of water and acetonitrile (containing 1% acetic acid) as eluent. The organic solvents were evaporated and the pH of the aqueous phase was adjusted to 7.5. The solution was extracted with methylene chloride and the organic phase was dried over magnesium sulphate and evaporated. The resultant residue was triturated under diethyl ether to give the required product in 69% yield which gave the following characterising data; NMR Spectrum: (CDCl₃) 2.0 (m, 2H), 2.3 (m, 2H), 2.4 (m, 2H), 2.3 (t, 2H), 3.3 (d, 2H), 3.55 (m, 2H), 4.1 (m, 2H), 4.3 (t, 2H), 4.65 (m, 2H), 4.8 (m, 1H), 4.9 (s, 1H), 5.2 (s, 1H), 6.2 (s, 2H), 6.6 (s, 1H), 6.9 (s, 1H), 7.8 (s, 1H), 8.65 (s, 1H), 9.5 (s, 1H); Mass Spectrum: M+H⁺ 558 and 560; Elemental Analysis: Found C, 56.5;
   H, 5.3; N, 12.5; C₂₆H₂₈ClN₅O₇ 0.2Et₂O requires C, 56.2; H, 5.3; N, 12.2%.
   The (3RS,4SR)-3,4-methylenedioxypyrrolidine used as a starting material was prepared as follows:-
   A solution of di-tert-butyl dicarbonate (Boc₂O, 78.95 g) in ethyl acetate (125 ml) was added dropwise to a stirred mixture of 3-pyroline (25 g; 65% pure containing pyrrolidine) and ethyl acetate (125 ml) which had been cooled to 0°C. The reaction temperature was maintained at 5-10°C during the addition. The resultant reaction mixture was allowed to warm to ambient temperature overnight. The reaction mixture was washed successively with water, 0.1N aqueous hydrochloric acid solution, water, a saturated aqueous sodium bicarbonate solution and brine, dried over magnesium sulphate and evaporated. There was thus obtained, as a colorless oil (62 g), a 2:1 mixture of tert-butyl 3-pyrroline-1-carboxylate, NMR: (CDCl₃) 1.45 (s, 9H), 4.1 (d, 4H), 6.75 (m, 2H), and tert-butyl pyrrolidine-1-carboxylate, NMR: (CDCl₃) 1.5 (s, 9H), 1.8 (br s, 4H), 3.3 (br s, 4H).
   A solution of the mixture of materials so obtained in acetone (500 ml) was added dropwise to a mixture of N-methylmorpholine-N-oxide (28.45 g), osmium tetroxide (1 g) and water (500 ml) whilst keeping the reaction temperature below 25°C. The reaction mixture was then stirred at ambient temperature for 5 hours. The solvent was evaporated and the residue was partitioned between ethyl acetate and water. The organic phase was washed with brine, dried over magnesium sulphate and evaporated. The residue was purified by column chromatography on silica using increasingly polar mixtures of petroleum ether (b.p. 40-60°C) and ethyl acetate as eluent and by further column chromatography on silica using increasingly polar mixtures of methylene chloride and methanol. There was thus obtained tert-butyl (3RS,4SR)-3,4-dihydroxypycrolidine-1-carboxylate as an oil (34.6 g); NMR Spectrum: (CDCl₃) 1.45 (s, 9H), 2.65 (m, 2H), 3.35 (m, 2H), 3.6 (m, 2H), 4.25 (m, 2H).
      A solution of tert-butyl (3RS,4SR)-3,4-dihydroxypyrrolidine-1-carboxylate (34.6 g) in DMF (400 ml) was cooled to 0-5°C and sodium hydride (60% dispersion in mineral oil, 0.375 mol) was added portionwise. The reaction mixture was stirred at 5°C for 1 hour. Dibromomethane (15.6 ml) was added and the reaction mixture was stirred at 5°C for 30 minutes. The reaction mixture was allowed to warm to ambient temperature and was stirred for 16 hours. The DMF was evaporated and the residue was partitioned between ethyl acetate and water. The organic phase was washed with water and with brine, dried over magnesium sulphate and evaporated. The residue was purified by column chromatography on silica using increasingly polar mixtures of petroleum ether (b.p. 40-60°C) and ethyl acetate as eluent. There was thus obtained tert-butyl (3RS,4SR)-3,4-methylenedioxypyrrolidine-1-carboxylate as a colourless oil (19.77 g);NMR Spectrum: (CDCl₃) 1.45 (s, 9H), 3.35 (m, 2H), 3.75 (br s, 2H), 4.65 (m, 2H), 4.9 (s, 1H), 5.1 (s, 1H).
      A cooled 5M solution of hydrogen chloride in isopropanol (150 ml) was added to a solution of tert-butyl (3RS,4SR)-3,4-methylenedioxypyrrolidine-1-carboxylate (19.7 g) in methylene chloride (500 ml) that was cooled in an ice bath. The reaction mixture was allowed to warm to ambient temperature and was stirred for 4 hours. The solvent was evaporated and the residue was triturated under diethyl ether. The precipitate was collected by filtration, washed with diethyl ether and dried. There was thus obtained (3RS,4SR)-3,4-methylenedioxypyrrolidine hydrochloride as a beige solid (13.18 g); NMR Spectrum: (DMSOd₆) 3.15 (m, 2H), 3.35 (m, 2H), 4.65 (s, 1H), 4.8 (m, 2H), 5.1 (s, 1H).
      The material so obtained was suspended in diethyl ether and a saturated methanolic ammonia solution was added. The resultant mixture was stirred at ambient temperature for 10 minutes. The mixture was filtered and the solvent was evaporated at ambient temperature under vacuum There was thus obtained (3RS,4SR)-3,4-methylenedioxypyrrolidine which was used without any additional purification.
[6] The reactants were 7-(2-chloroethoxy)-4-(5-chloro-2,3-methylenedioxypyrid-4-ylamino)-5-isopropoxyquinazoline and 1-acetylpiperazine. The reaction mixture was heated to 85°C for 8 hours. The reaction product was purified by column chromatography on silica using increasingly polar mixtures of methylene chloride and methanol as eluent. The product was obtained in 89% yield and gave the following characterising data; m.p. 208-210°C; NMR Spectrum: (CDCl₃) 1.55 (d, 6H), 2.1 (s, 3H), 2.6 (m, 4H), 2.9 (t, 2H), 3.5 (t, 2H), 3.7 (t, 2H), 4.25 (t, 2H), 4.85 (m, 1H), 6.15 (s, 2H), 6.55 (s, 1H), 6.85 (s, 1H), 7.75 (s, 1H), 8.6 (s, 1H), 9.6 (s, 1H); Mass Spectrum: M+H⁺ 529 and 531; Elemental Analysis: Found C, 57.0; H, 5.7; N, 15.7; C₂₅H₂₉ClN₆O₅ requires C, 56.8; H, 5.5; N, 15.9%.
[7] The reactants were 7-(2-chloroethoxy)-4-(5-chloro-2,3-methylenedioxypyrid-4-ylamino)-5-isopropoxyquinazoline and (3RS,4SR)-3,4-methylenedioxypyrrolidine. The reaction mixture was heated to 95°C for 3 hours. The reaction product was purified by column chromatography on a C18 reversed phase silica column (Waters Symmetry column, 5 microns silica, 19 mm diameter, 100 mm length) using a decreasingly polar mixture of water and acetonitrile (containing 1% acetic acid) as eluent. The organic solvents were evaporated and the pH of the aqueous phase was adjusted to 7. The solution was extracted with methylene chloride and the organic phase was dried over magnesium sulphate and evaporated. The resultant residue was triturated under diethyl ether to give the required product in 64% yield which gave the following characterising data; NMR Spectrum: (CDCl₃) 1.55 (d, 6H), 2.35 (m, 2H), 2.9 (t, 2H), 3.25 (d, 2H), 4.25 (t, 2H), 4.6 (m, 2H), 4.85 (m, 1H), 4.9 (s, 1H), 5.15 (s, 1H), 6.15 (s, 2H), 6.55 (s, 1H), 6.85 (s, 1H), 7.75 (s, 1H), 8.6 (s, 1H), 9.6 (s, 1H); Mass Spectrum: M+H⁺ 516 and 518; Elemental Analysis: Found C, 54.7; H, 5.2; N, 13.2; C₂₄H₂₆ClN₅O₆ 0.5H₂O requires C, 54.9; H, 5.2; N, 13.3%.
[8] The reactants were 4-(5-chloro-2,3-methylenedioxypyrid-4-ylamino)-6-(2-chloroethoxy)-7-methoxyquinazoline (the preparation of which is described in Example 7 hereinafter) and morpholine. The reaction mixture was heated to 120°C for 16 hours. The required product was obtained in 69% yield and gave the following characterising data; NMR Spectrum: (CDCl₃ and CD₃CO₂D) 3.3 (m, 4H), 3.5 (t, 2H), 3.95 (m, 4H), 4.05 (s, 3H), 4.6 (t, 2H), 6.15 (s, 2H), 7.6 (s, 1H), 7.8 (s, 2H), 8.6 (s, 1H); Mass Spectrum: M+H⁺ 460 and 462; Elemental Analysis: Found C, 53.45; H, 4.8; N, 14.5; C₂₁H₂₂ClN₅O₅ 0.55H₂O requires C, 53.7; H, 5.0; N, 14.9%.
[9] The reactants were 4-(5-chloro-2,3-methylenedioxypyrid-4-ylamino)-6-(2-chloroethoxy)-7-methoxyquinazoline and 1-methylpiperazine. The reaction mixture was heated to 120°C for 16 hours. The reaction product was purified by column chromatography on a Waters X-Terra silica column (C 18 reversed-phase, 5 microns, 19 mm diameter, 100 mm length; Waters Inc., Milford, MA01757, USA) and eluted with decreasingly polar mixtures of an ammonium carbonate buffer (2 g/L in water) and acetonitrile. Appropriate fractions were collected, the organic solvent was evaporated and the resultant mixture was partitioned between ethyl acetate and a saturated aqueous sodium bicarbonate solution. The organic phase was dried over magnesium sulphate and evaporated. There was thus obtained the required product in 29% yield which gave the following characterising data; NMR Spectrum: (CDCl₃ and CD₃CO₂D) 2.7 (s, 3H), 3.25-3.35 (br m, 10H), 4.05 (s, 3H), 4.45 (t, 2H), 6.15 (s, 2H), 7.55 (s, 1H), 7.7 (s, 1H), 7.8 (s, 1H), 8.65 (s, 1H); Mass Spectrum: M+H⁺ 473 and 475; Elemental Analysis: Found C, 54.9; H, 5.3; N, 17.1; C₂₂H₂₅ClN₆O₄ 0.4H₂O requires C, 55.0; H, 5.4; N, 17.5%.
[10] The reactants were 4-(5-chloro-2,3-methylenedioxypyrid-4-ylamino)-6-(2-chloroethoxy)-7-methoxyquinazoline and pyrrolidine. The reaction mixture was heated to 120°C for 16 hours. The required product was obtained in 41 % yield and gave the following characterising data; NMR Spectrum: (CDCl₃ and CD₃CO₂D) 2.15 (m, 4H), 3.3-3.6 (br s, 4H), 3.7 (t, 2H), 4.05 (s, 3H), 4.65 (t, 2H), 6.15 (s, 2H), 7.65 (s, 1H), 7.8 (s, 1H), 7.9 (s, 1H), 8.65 (s, 1H); Mass Spectrum: M+H⁺ 444 and 446; Elemental Analysis: Found C, 55.0; H, 5.0; N, 14.9; C₂₁H₂₂ClN₅O₄ 0.7H₂O requires C, 55.25; H, 5.2; N, 15.3%.
[11) The reactants were 4-(5-chloro-2,3-methylenedioxypyrid-4-ylamino)-6-(2-chloroethoxy)-7-methoxyquinazoline and 1-acetylpiperazine. The reaction mixture was heated to 120°C for 16 hours. The required product was obtained in 51 % yield and gave the following characterising data; NMR Spectrum: (CDCl₃ and CD₃CO₂D) 2.15 (s, 3H), 3.1 (m, 2H), 3.2 (m, 2H), 3.4 (t, 2H), 3.75 (m, 2H), 3.85 (m, 2H), 4.0 (s, 3H), 4.55 (t, 2H), 6.15 (s, 2H), 7.6 (s, 1H), 7.7 (s, 1H), 7.8 (s, 1H), 8.6 (s, 1H); Mass Spectrum: M+H⁺ 501 and 503.
[12] The reactants were 4-(5-chloro-2,3-methylenedioxypyrid-4-ylamino)-6-(2-chloroethoxy)-7-methoxyquinazoline and (3RS,4SR)-3,4-methylenedioxypyrrolidine. The reaction mixture was heated to 120°C for 16 hours. The required product was obtained in 73% yield and gave the following characterising data; NMR Spectrum: (CDCl₃ and CD₃CO₂D) 2.95 (m, 2H), 3.45 (t, 2H), 3.65 (d, 2H), 4.05 (s, 3H), 4.55 (t, 2H), 4.8 (m, 3H), 5.2 (s, 1H), 6.15 (s, 2H), 7.6 (s, 1H), 7.75 (s, 1H), 7.8 (s, 1H), 8.65 (s, 1H); Mass Spectrum: M+H⁺ 488 and 490.
[13] The reactants were 4-(5-chloro-2,3-methylenedioxypyrid-4-ylamino)-6-(3-chloropropoxy)-7-methoxyquinazoline (the preparation of which is described in Example 8 hereinafter) and pyrrolidine. The reaction mixture was heated to 120°C for 16 hours. The required product was obtained in 50% yield and gave the following characterising data; NMR Spectrum: (CDCl₃ and CD₃CO₂D) 2.1 (m, 4H), 2.4 (m, 2H), 3.0-3.8 (br s, 4H), 3.4 (t, 2H), 4.05 (s, 3H), 4.35 (t, 3H), 6.1 (s, 2H), 7.6 (s, 1H), 7.75 (s, 1H), 7.8 (s, 1H), 8.65 (s, 1H); Mass Spectrum: M+H⁺ 458 and 460; Elemental Analysis: Found C, 57.3; H, 5.4; N, 14.5; C₂₂H₂₄ClN₅O₄ 0.15H₂O requires C, 57.4; H, 5.3; N, 15.2%.
[14] The reactants were 4-(5-chloro-2,3-methylenedioxypyrid-4-ylamino)-6-(3-chloropropoxy)-7-methoxyquinazoline and morpholine. The reaction mixture was heated to 120°C for 16 hours. The required product was obtained in 72% yield and gave the following characterising data; NMR Spectrum: (CDCl₃) 2.1 (m, 2H), 2.5 (m, 4H), 2.6 (t, 2H), 3.7 (m, 4H), 4.05 (s, 3H), 4.25 (t, 2H), 6.1 (s, 2H), 7.05 (s, 1H), 7.15 (s, 1H), 7.3 (s, 1H), 7.75 (s, 1H), 8.7 (s, 1H); Mass Spectrum: M+H⁺ 474 and 476.
[15] The reactants were 4-(5-chloro-2,3-methylenedioxypyrid-4-ylamino)-6-(3-chloropropoxy)-7-methoxyquinazoline and 1-acetylpiperazine. The reaction mixture was heated to 120°C for 16 hours. The required product was obtained in 39% yield and gave the following characterising data; NMR Spectrum: (CDCl₃ and CD₃CO₂D) 2.15 (s, 3H), 2.35 (m, 2H), 3.15-3.3 (m, 6H), 3.8 (m, 2H), 3.9 (m, 2H), 4.0 (s, 3H), 4.3 (t, 2H), 6.15 (s, 2H), 7.6 (s, 1H), 7.65 (s, 1H), 7.8 (s, 1H), 8.65 (s, 1H); Mass Spectrum: M+H⁺ 515 and 517.
[16] The reactants were 4-(5-chloro-2,3-methylenedioxypyrid-4-ylamino)-6-(3-chloropropoxy)-7-methoxyquinazoline and 1-acetylpiperazine. The reaction mixture was heated to 120°C for 16 hours. The required product was obtained in 27% yield and gave the following characterising data; NMR Spectrum: (CDCl₃ and CD₃CO₂D) 2.3 (m, 2H), 2.7 (s, 3H), 3.3 (t, 2H), 3.4 (m, 4H), 3.5 (m, 4H), 4.0 (s, 3H), 4.3 (t, 2H), 6.15 (s, 2H), 7.6 (s, 1H), 7.65 (s, 1H), 7.8 (s, 1H), 8.65 (s, 1H); Mass Spectrum: M+H⁺487 and 489.
[17] The reactants were 4-(5-chloro-2,3-methylenedioxypyrid-4-ylamino)-6-(3-chloropropoxy)-7-methoxyquinazoline and (3RS,4SR)-3,4-methylenedioxypyrrolidine. The reaction mixture was heated to 95°C for 3 hours. The reaction product was purified by column chromatography on a C18 reversed phase silica column (Waters Symmetry column, 5 microns silica, 19 mm diameter, 100 mm length) using a decreasingly polar mixture of water and acetonitrile (containing 1% acetic acid) as eluent. The organic solvents were evaporated and the pH of the aqueous phase was adjusted to 7. The solution was extracted with methylene chloride and the organic phase was dried over magnesium sulphate and evaporated. The resultant residue was triturated under diethyl ether to give the required product in 57% yield which gave the following characterising data; NMR Spectrum: (CDCl₃ and CD₃CO₂D) 2.3 (m, 2H), 3.3 (m, 2H), 3.4 (t, 2H), 3.6 (d, 2H), 4.0 (s, 3H), 4.3 (t, 2H), 4.8 (m, 3H), 5.2 (s, 1H), 6.15 (s, 2H), 7.55 (s, 1H), 7.6 (s, 1H), 7.8 (s, 1H), 8.6 (s, 1H); Mass Spectrum: M+H⁺ 502 and 504.
[18] The reactants were 4-(5-chloro-2,3-methylenedioxypyrid-4-ylamino)-7-(2-chloroethoxy)-5-tetrahydropyran-4-yloxyquinazoline and 1-prop-2-ynylpiperazine. The reaction mixture was heated to 80°C for 3 hours and then to 110°C for 5 hours. The reaction product was purified by column chromatography on a Waters X-Terra silica column (C18 reversed-phase, 5 microns, 19 mm diameter, 100 mm length) and eluted with decreasingly polar mixtures of an ammonium carbonate buffer (2 g/L in water) and acetonitrile. Appropriate fractions were collected, the organic solvent was evaporated and the resultant mixture was partitioned between ethyl acetate and a saturated aqueous sodium bicarbonate solution. The organic phase was dried over magnesium sulphate and evaporated. There was thus obtained the required product in 54% yield which gave the following characterising data; NMR Spectrum: (DMSOd₆ and CD₃CO₂D) 1.85 (m, 2H), 2.15 (m, 2H), 2.5-3.0 (m, 10H), 3.15 (s, 1H), 3.3 (s, 2H), 3.55 (t, 2H), 3.9 (m, 2H), 4.3 (m, 2H), 5.05 (m, 1H), 6.2 (s, 2H), 6.9 (s, 2H), 7.8 (s, 1H), 8.5 (s, 1H); Mass Spectrum: M+H⁺ 567 and 569; Elemental Analysis: Found C, 55.9; H, 5.6; N, 14.0; C₂₈H₃₁ClN₆O5 2H₂O requires C, 55.8; H, 5.85; N, 13.9%.
[19] Using the detailed conditions described in Note [ 18] immediately above, 4-(5-chloro-2,3-methylenedioxypyrid-4-ylamino)-7-(2-chloroethoxy)-5-tetrahydropyran-4-yloxyquinazoline was reacted with morpholine to give the required product in 48% yield which gave the following characterising data; NMR Spectrum: (DMSOd₆ and CD₃CO₂D) 1.8 (m, 2H), 2.15 (m, 2H), 2.55 (m, 4H), 2.8 (m, 2H), 3.5 (m, 2H), 3.6 (m, 4H), 3.9 (m, 2H), 4.3 (t, 2H), 5.1 (m, 1H), 6.2 (s, 2H), 6.9 (m, 2H), 7.8 (s, 1H), 8.45 (s, 1H); Mass Spectrum: M+H⁺ 530 and 532; Elemental Analysis: Found C, 51.8; H, 5.8; N, 12.1; C₂₅H₂₈ClN₅O₆ 2.5H₂O requires C, 52.2; H, 5.8; N, 12.2%.
[20] The reactants were 4-(5-chloro-2,3-methylenedioxypyrid-4-ylamino)-7-(3-chloropropoxy)-5-tetrahydropyran-4-yloxyquinazoline (described in Example 9 hereinafter) and morpholine. The required product was obtained in 30% yield and gave the following characterising data; NMR Spectrum: (CDCl₃ and CF₃CO₂D) 2.05 (m, 2H), 2.35 (m, 4H), 3.15 (m, 2H), 3.45 (m, 2H), 3.75 (m, 4H), 3.9 (m, 2H), 4.2 (m, 6H), 5.0 (m, 1H), 6.3 (s, 2H), 6.85 (s, 1H), 7.0 (s, 1H), 7.9 (s, 1H), 8.7 (s, 1H); Mass Spectrum: M+H⁺ 544 and 546.
[21] The reactants were 4-(5-chloro-2,3-methylenedioxypyrid-4-ylamino)-7-(3-chloropropoxy)-5-tetrahydropyran-4-yloxyquinazoline and 1-prop-2-ynylpiperazine. The reaction product was purified by column chromatography on a C18 reversed phase silica column (Waters Symmetry column, 5 microns silica, 19 mm diameter, 100 mm length) using a decreasingly polar mixture of water and acetonitrile (containing 1% acetic acid) as eluent. The organic solvents were evaporated and the pH of the aqueous phase was adjusted to 9. The solution was extracted with methylene chloride and the organic phase was dried over magnesium sulphate and evaporated. The resultant residue was triturated under pentane to give the required product in 48% yield which gave the following characterising data; NMR Spectrum: (DMSOd₆ and CD₃CO₂D) 1.85 (m, 2H), 2.0 (m, 2H), 2.15 (m, 2H), 2.5-2.8 (br m, 10H), 3.15 (s, 1H), 3.3 (s, 2H), 3.55 (t, 2H), 3.9 (m, 2H), 4.2 (t, 2H), 5.05 (m, 1H), 6.2 (s, 2H), 6.85 (s, 1H), 6.9 (s, 1H), 7.8 (s, 1H), 8.45 (s, 1H); Mass Spectrum: M+H⁺ 581 and 583.
[22] The reactants were 7-(2-chloroethoxy)-4-(5-chloro-2,3-methylenedioxypyrid-4-ylamino)-5-isopropoxyquinazoline and piperazine. The required product was obtained in 30% yield and gave the following characterising data; NMR Spectrum: (CDCl₃) 1.55 (d, 6H), 2.6 (m, 4H), 2.85 (t, 2H), 2.95 (m, 4H), 4.25 (t, 2H), 4.85 (m, 1H), 6.15 (s, 2H), 6.55 (s, 1H), 6.85 (s, 1H), 7.75 (s, 1H), 8.6 (s, 1H), 9.6 (s, 1H); Mass Spectrum: M+H⁺ 487 and 489; Elemental Analysis: Found C, 55.4; H, 5.5; N, 16.4; C₂₃H₂₇ClN₆O₄ 0.1Et₂O 0.6H₂O requires C, 55.65; H, 5.8; N, 16.6%.
[23] The reactants were 7-(2-chloroethoxy)-4-(5-chloro-2,3-methylenedioxypyrid-4-ylamino)-5-isopropoxyquinazoline and 1-(2-hydroxyethyl)piperazine. The reaction mixture was heated to 85°C for 8 hours. The reaction product was purified by column chromatography on silica using increasingly polar mixtures of methylene chloride and methanol as eluent. The material so obtained was triturated under diethyl ether to give the required product in 67% yield which gave the following characterising data; NMR Spectrum: (CDCl₃) 1.5 (d, 6H), 2.5-2.7 (br m, 12H), 3.65 (t, 2H), 4.25 (t, 2H), 4.8 (m, 1H), 6.15 (s, 2H), 6.6 (s, 1H), 6.85 (s, 1H), 7.25 (s, 1H), 7.75 (s, 1H), 8.6 (s, 1H), 9.6 (s, 1H); Mass Spectrum: M+H⁺ 531 and 533; Elemental Analysis: Found C, 55.4; H, 6.05; N, 15.2; C₂₅H₃₁ClN₆O₅ 0.1Et₂O 0.5H₂O requires C, 55.7; H, 6.1; N, 15.35%.
[24] The reactants were 7-(2-chloroethoxy)-4-(5-chloro-2,3-methylenedioxypyrid-4-ylamino)-5-isopropoxyquinazoline and pyrrolidine. The reaction mixture was heated to 80°C for 4 hours. The reaction product was purified by column chromatography on a C18 reversed phase silica column (Waters Symmetry column, 5 microns silica, 19 mm diameter, 100 mm length) using a decreasingly polar mixture of water and acetonitrile (containing 1% acetic acid) as eluent. The organic solvents were evaporated and the pH of the aqueous phase was adjusted to 9. The solution was extracted with methylene chloride and the organic phase was dried over magnesium sulphate and evaporated. The resultant residue was triturated under pentane to give the required product in 62% yield which gave the following characterising data; NMR Spectrum: (CDCl₃) 1.55 (d, 6H), 1.85 (m, 4H), 2.6 (m, 4H), 2.95 (t, 2H), 4.25 (t, 2H), 4.85 (m, 1H), 6.15 (s, 2H), 6.6 (s, 1H), 6.85 (s, 1H), 7.75 (s, 1H), 8.6 (s, 1H), 9.6 (s, 1H); Mass Spectrum: M+H⁺ 472 and 474; Elemental Analysis: Found C, 58.3; H, 5.4; N, 14.7; C₂₃H₂₆ClN₅O₄ requires C, 58.5; H, 5.55; N, 14.8%.
[25] Using the detailed conditions described in Note [24] immediately above, 4-(5-chloro-2,3-methylenedioxypyrid-4-ylamino)-7-(2-chloroethoxy)-5-tetrahydropyran-4-yloxyquinazoline was reacted with piperidine to give the required product in 52% yield which gave the following characterising data; NMR Spectrum: (CDCl₃) 1.45 (m, 2H), 1.55 (d, 6H), 1.65 (m, 4H), 2.5 (m, 4H), 2.85 (t, 2H), 4.25 (t, 2H), 4.85 (m, 1H), 6.15 (s, 2H), 6.6 (s, 1H), 6.85 (s, 1H), 7.75 (s, 1H), 8.6 (s, 1H), 9.6 (s, 1H); Mass Spectrum: M+H⁺ 486 and 488; Elemental Analysis: Found C, 59.3; H, 5.9; N, 14.4; C₂₄H₂₈ClN₅O₄ requires C, 59.3; H, 5.8; N, 14.4%.
[26] Using the detailed conditions described in Note [24] immediately above, 4-(5-chloro-2,3-methylenedioxypyrid-4-ylamino)-7-(2-chloroethoxy)-5-tetrahydropyran-4-yloxyquinazoline was reacted with morpholine to give the required product in 57% yield which gave the following characterising data; NMR Spectrum: (CDCl₃) 1.55 (d, 6H), 2.6 (m, 4H), 2.85 (t, 2H), 3.75 (m, 4H), 4.25 (t, 2H), 4.85 (m, 1H), 6.15 (s, 2H), 6.55 (s, 1H), 6.85 (s, 1H), 7.75 (s, 1H), 8.6 (s, 1H), 9.6 (s, 1H); Mass Spectrum: M+H⁺ 488 and 490; Elemental Analysis: Found C, 56.6; H, 5.4; N, 14.2; C₂₃H₂₆ClN₅O₅ requires C, 56.6; H, 5.4; N, 14.35%.
[27] Using the detailed conditions described in Note [24] immediately above, 4-(5-chloro-2,3-methylenedioxypyrid-4-ylamino)-7-(2-chloroethoxy)-5-tetrahydropyran-4-yloxyquinazoline was reacted with 1-prop-2-ynylpiperazine to give the required product in 41% yield which gave the following characterising data; NMR Spectrum: (CDCl₃) 1.55 (d, 6H), 2.25 (s, 1H), 2.65 (br m, 8H), 2.9 (t, 2H), 3.3 (s, 2H), 4.25 (t, 2H), 4.85 (m, 1H), 6.15 (s, 2H), 6.55 (s, 1H), 6.85 (s, 1H), 7.75 (s, 1H), 8.6 (s, 1H), 9.6 (s, 1H); Mass Spectrum: M+H⁺ 525 and 527; Elemental Analysis: Found C, 59.3; H, 5.4; N, 15.85; C₂₆H₂₉ClN₆O₄ requires C, 59.5; H, 5.6; N, 16.0%.
[28] The reactants were 7-(2-chloroethoxy)-4-(5-chloro-2,3-methylenedioxypyrid-4-ylamino)-5-isopropoxyquinazoline and (3RS,4SR)-3,4-dimethoxypyrrolidine. The required product was obtained in 78% yield and gave the following characterising data; NMR Spectrum: (DMSOd₆ and CD₃CO₂D) 1.45 (d, 6H), 2.7 (m, 2H), 3.0 (m, 2H), 3.15 (m, 2H), 3.3 (s, 6H), 3.75 (m, 2H), 4.25 (t, 2H), 5.5 (m, 1H), 6.2 (s, 2H), 6.8 (s, 1H), 6.85 (s, 1H), 7.8 (s, 1H), 8.45 (s, 1H); Mass Spectrum: M+H⁺ 532 and 534; Elemental Analysis: Found C, 56.0; H, 5.6; N, 12.85; C₂₅H₃₀ClN₅O₆ 0.3H₂O requires C, 56.25; H, 5.7; N, 13.1 %.
   The (3RS,4SR)-3,4-dimethoxypyrrolidine used as a starting material was obtained as follows:-
   A solution of tert-butyl (3RS,4SR)-3,4-dihydroxypyrrolidine-1-carboxylate (1g) in DMF (20 ml) was cooled to 0-5°C and sodium hydride (60% dispersion in mineral oil, 0.433 g) was added portionwise. The reaction mixture was stirred at 5°C for 1 hour. Methyl iodide (0.675 ml) was added and the reaction mixture was allowed to warm to ambient temperature and was stirred for 16 hours. The DMF was evaporated and the residue was partitioned between diethyl ether and water. The organic phase was washed with water and with brine, dried over magnesium sulphate and evaporated. The residue was purified by column, chromatography on silica using increasingly polar mixtures of petroleum ether (b.p. 40-60°C) and ethyl acetate as eluent. There was thus obtained tert-butyl (3RS,4SR)-3,4-diinethoxypyrrolidine-1-carboxylate as an oil (1.06 g); NMR Spectrum: (CDCl₃) 1.45 (s, 9H), 3.35 (m, 1H), 3.45 (s, 6H), 3.5 (m, 2H), 3.55 (m, 1H), 3.85 (m, 2H).
   A cooled 5M solution of hydrogen chloride in isopropanol (3 ml) was added to a solution of tert-butyl (3RS,4SR)-3,4-dimethoxypyrrolidine-1-carboxylate (1 g) in methylene chloride (25 ml) that was cooled in an ice bath. The reaction mixture was allowed to warm to ambient temperature and was stirred for 16 hours. The solvent was evaporated. There was thus obtained (3RS,4SR)-3,4-dimethoxypyrrolidine hydrochloride as an oil (0.72 g); NMR Spectrum: (DMSOd₆) 3.1 (m, 2H), 3.25 (m, 2H), 3.35 (s, 6H), 4.0 (m, 2H), 9.3 (br s, 1H), 9.5 (br s, 1H).
   The material so obtained was dissolved in methylene chloride and a 7M methanolic ammonia solution (0.2 ml) was added. The resultant mixture was stirred at ambient temperature for 5 minutes. The mixture was filtered and the solvent was evaporated at ambient temperature under vacuum There was thus obtained (3RS,4SR)-3,4-dimethoxypyrrolidine which was used without any additional purification.
[29] Using the detailed conditions described in Note [24] immediately above except that the product was triturated under diethyl ether rather than under pentane, 4-(5-chloro-2,3-methylenedioxypyrid-4-ylamino)-7-(2-chloroethoxy)-5-tetrahydropyran-4-yloxyquinazoline was reacted with (3RS,4SR)-3,4-ethylidenedioxypyrrolidine to give the required product in 67% yield which gave the following characterising data; NMR Spectrum (CDCl₃) 1.45 (d, 3H), 1.55 (d, 6H), 2.3 (d, 2H), 2.95 (m, 2H), 3.25 (d, 2H), 4.25 (t, 2H), 4.55 (m, 2H), 4.8 (m, 1H), 5.0 (m, 1H), 6.15 (s, 2H), 6.55 (s, 1H), 6.85 (s, 1H), 7.75 (s, 1H), 8.6 (s, 1H), 9.6 (s, 1H); Mass Spectrum: M+H⁺ 530 and 532; Elemental Analysis: Found C, 56.7; H, 5.5; N, 12.9; C₂₅H₂₈ClN₅O₆ 0.1Et₂O requires C, 56.8; H, 5.4; N, 13.0%.
   The (3RS,4SR)-3,4-ethylidenedioxypyrrolidine used as a starting material was obtained as follows:-
   A solution of tert-butyl (3RS,4SR)-3,4-dihydroxypyrrolidine-1-carboxylate (0.5 g) in methylene chloride (15 ml) was cooled to 0-5°C and acetaldehyde dimethylacetal (0.782 ml) and 4-toluenesulphonic acid (0.025 g) were added in turn. The reaction mixture was stirred at ambient temperature for 2 hours. The resultant mixture was evaporated and the residue was purified by column chromatography on silica using increasingly polar mixtures of petroleum ether (b.p. 40-60°C) and ethyl acetate as eluent. There was thus obtained tert-butyl (3RS,4SR)-3,4-ethylidenedioxypyrrolidine-1-carboxylate as an oil (0.484 g);NMR Spectrum: (CDCl₃) 1.4 (d, 3H), 1.45 (s, 9H), 3.3 (m, 2H), 3.8 (m, 2H), 4.6 (m, 2H), 5.0 (q, 1H).
      A cooled 5M solution of hydrogen chloride in isopropanol (4 ml) was added to a solution of tert-butyl (3RS,4SR)-3,4-ethylidenedioxypyrrolidine-1-carboxylate (0.475 g) in methylene chloride (25 ml) that was cooled in an ice bath. The reaction mixture was allowed to warm to ambient temperature and was stirred for 2 hours. The solvent was evaporated and the residue was triturated under diethyl ether. The precipitate was collected by filtration, washed with diethyl ether and dried. There was thus obtained (3RS,4SR)-3,4-ethylidenedioxypyrrolidine hydrochloride (0.28 g); NMR Spectrum: (DMSOd₆ and CD₃CO₂D) 1.35 (d, 3H), 3.1 (d, 2H), 3.4 (d, 2H), 4.75 (s, 2H), 4.9 (q, 1H).
      The material so obtained was dissolved in methylene chloride and a 7M methanolic ammonia solution (0.2 ml) was added. The resultant mixture was stirred at ambient temperature for 5 minutes. The mixture was filtered and the solvent was evaporated at ambient temperature under vacuum There was thus obtained (3RS,4SR)-3,4-ethylidenedioxypyrrolidine which was used without any additional purification.
[30] The reactants were 7-(2-chloroethoxy)-4-(5-chloro-2,3-methylenedioxypyrid-4-ylamino)quinazoline and 1-methylpiperazine. The required product was obtained in 74% yield and gave the following characterising data; NMR Spectrum: (CDCl₃ and CD₃CO₂D); Mass Spectrum: M+H⁺ 501 and 503; Elemental Analysis: Found C, 57.5; H, 6.5; N, 16.0; C₂₄H₂₉ClN₆O₄ 0.23H₂O requires C, 57.8; H, 6.1; N, 16.2%.
[31] The reactants were 7-(3-chloropropoxy)-4-(5-chloro-2,3-methylenedioxypyrid-4-ylamino)-5-isopropoxyquinazoline (the preparation of which is described in Example 12 hereinafter) and morpholine. The required product was obtained in 39% yield and gave the following characterising data; NMR Spectrum: (CDCl₃) 1.55 (d, 6H), 2.05 (m, 2H), 2.45 (m, 4H), 2.55 (t, 2H), 3.7 (m, 4H), 4.15 (t, 2H), 4.85 (m, 1H), 6.15 (s, 2H), 6.5 (s, 1H), 6.85 (s, 1H), 7.75 (s, 1H), 8.6 (s, 1H), 9.6 (s, 1H); Mass Spectrum: M+H⁺ 502 and 504; Elemental Analysis: Found C, 57.3; H, 5.65; N, 13.6; C₂₄H₂₈ClN₅O₅ requires C, 57.4; H, 5.6; N, 13.95%.
[32] The reactants were 7-(3-chloropropoxy)-4-(5-chloro-2,3-methylenedioxypyrid-4-ylamino)quinazoline (the preparation of which is described in Example 13 hereinafter) and morpholine. The required product was obtained in 45% yield and gave the following characterising data; NMR Spectrum (DMSOd₆ and CF₃CO₂D) 2.3 (m, 2H), 3.15 (m, 2H), 3.35 (m, 2H), 3.5 (m, 2H), 3.7 (m, 2H), 4.05 (m, 2H), 4.35 (m, 2H), 6.3 (s, 2H), 7.35 (s, 1H), 7.6 (d, 1H), 7.9 (s, 1H), 8.7 (d, 1H), 9.05 (s, 1H); Mass Spectrum M+H⁺ 444 and 446; Elemental Analysis: Found C, 57.0; H, 5.1; N, 15.7; C₂₁H₂₂ClN₅O₄ requires C, 56.8; H, 5.0; N, 15.8%.
[33] The reactants were 7-(3-chloropropoxy)-4-(5-chloro-2,3-methylenedioxypyrid-4-ylamino)quinazoline and 1-acetylpiperazine. The required product was obtained in 34% yield and gave the following characterising data; NMR Spectrum (DMSOd₆ and CF₃CO₂D) 2.05 (s, 3H), 2.3 (s, 2H), 3.0 (m, 2H), 3.15 (m, 1H), 3.3-3.4 (m, 4H), 3.6 (m, 2H), 4.05 (m, 1H), 4.35 (m, 2H), 4.5 (m, 1H), 6.3 (s, 2H), 7.35 (s, 1H), 7.6 (d, 1H), 7.9 (s, 1H), 8.7 (d, 1H), 9.0 (s, 1H); Mass Spectrum: M+H⁺ 485 and 487; Elemental Analysis: Found C, 56.9; H, 5.4; N, 16.6; C₂₃H₂₅ClN₆O₄ 0.15Et₂O requires C, 57.1; H, 5.4; N, 16.9%.
[34] The reactants were 7-(2-chloroethoxy)-4-(2,3-methylenedioxypyrid-4-ylamino)quinazoline (the preparation of which is described in Example 14 hereinafter) and 1-prop-2-ynylpiperazine. After cooling of the reaction mixture and evaporation of the solvent, the residue was triturated under water and the resultant precipitate was isolated, washed with water and diethyl ether and dried. The required product was obtained in 60% yield and gave the following characterising data; NMR Spectrum: (CDCl₃) 2.26 (s, 1H), 2.8-2.6 (m, 8H), 2.97 (t, 2H), 3.3 (s, 2H); 4.03 (s, 3H), 4.33 (t, 2H), 6.14 (s, 2H), 6.98 (s, 1H), 7.12 (br s, 1H), 7.30 (s, 1H), 7.73 (d, 1H), 8.08 (d, 1H), 8.76 (s, 1H); Mass Spectrum: M+H⁺ 463.
[35] The reactants were 7-(3-chloropropoxy)-4-(2,3-methylenedioxypyrid-4-ylamino)quinazoline (the preparation of which is described in Example 15 hereinafter) and 1-prop-2-ynylpiperazine. The required product was obtained in 57% yield and gave the following characterising data; NMR Spectrum: (CDCl₃) 2.13 (m, 2H), 2.26 (s, 1H), 2.6 (m, 10H), 3.31 (s, 2H), 4.04 (s, 3H), 4.26 (t, 2H), 6.14 (s, 2H), 6.98 (s, 1H), 7.12 (br s, 1H), 7.31 (s, 1H), 7.72 (d, 1H), 8.08 (d, 1H), 8.76 (s, 1H); Mass Spectrum: M+H⁺477.

### Example 7

### 6-(2-chloroethoxy)-4-(5-chloro-2,3-methylenedioxypyrid-4-ylamino)-7-methoxyquinazoline

Using an analogous procedure to that described in Example 1, 4-chloro-6-(2-chloroethoxy)-7-methoxyquinazoline was reacted with 4-amino-5-chloro-2,3-methylenedioxypyridine to give the title compound in 59% yield; NMR Spectrum: (CDCl₃) 3.95 (t, 2H), 4.05 (s, 3H), 4.4 (t, 2H), 6.1 (s, 2H), 7.05 (s, 1H), 7.2 (s, 1H), 7.35 (s, 1H), 7.75 (s, 1H), 8.75 (s, 1H); Mass Spectrum: M+H⁺ 409 and 411.

The 4-chloro-6-(2-chloroethoxy)-7-methoxyquinazoline used as a starting material was prepared as follows:-
A mixture of 6-acetoxy-7-methoxy-3,4-dihydroquinazolin-4-one (International Patent Application WO 96/15118, Example 39 thereof; 8 g), thionyl chloride (80 ml) and DMF (0.8 ml) was stirred and heated to 80°C for 1.5 hours. The mixture was cooled to ambient temperature and the thionyl chloride was evaporated. The material so obtained was suspended in toluene and evaporated to dryness (twice). The resultant residue was diluted with methylene chloride (5 ml) and a 10:1 mixture (290 ml) of methanol and a saturated aqueous ammonium hydroxide solution was added. The resultant mixture was stirred and heated to 80°C for 5 minutes. The solvent was evaporated and the solid residue was suspended in water. The basicity of the mixture was adjusted to pH7 by the addition of dilute aqueous hydrochloric acid solution. The resultant solid was collected by filtration, washed with water and dried under vacuum over phosphorus pentoxide. There was thus obtained 4-chloro-6-hydroxy-7-methoxyquinazoline (6.08 g) which was used without further purification; NMR Spectrum: (DMSOd₆) 4.05 (s, 3H), 7.4 (s, 1H), 7.45 (s, 1H), 8.8 (s, 1H).

Di-tert-butyl azodicarboxylate (1.53 ml) was added portionwise over a few minutes to a stirred mixture of 4-chloro-6-hydroxy-7-methoxyquinazoline (1 g), 2-chloroethanol (0.382 ml), triphenylphosphine (1.74 g) and methylene chloride (30 ml) and the reaction mixture was stirred at ambient temperature for 2 hours. The mixture was evaporated and the residue was purified by column chromatography on silica using increasingly polar mixtures of methylene chloride and ethyl acetate as eluent. There was thus obtained 4-chloro-6-(2-chloroethoxy)-7-methoxyquinazoline as a white solid (1.06 g); NMR Spectrum: (CDCl₃) 3.95 (t, 2H), 4.05 (s, 3H), 4.45 (t, 2H), 7.35 (s, 1H), 7.4 (s, 1H), 8.9 (s, 1H).

### Example 8

### 6-(3-chloropropoxy)-4-(5-chloro-2,3-methylenedioxypyrid-4-ylamino)-7-methoxyquinazoline

Using an analogous procedure to that described in Example 1, 4-chloro-6-(3-chloropropoxy)-7-methoxyquinazoline was reacted with 4-amino-5-chloro-2,3-methylenedioxypyridine to give the title compound in 58% yield; NMR Spectrum: (CDCl₃) 2.4 (m, 2H), 3.8 (t, 2H), 4.05 (s, 3H), 4.35 (t, 2H), 6.15 (s, 2H), 7.05 (s, 1H), 7.2 (s, 1H), 7.3 (s, 1H), 7.75 (s, 1H), 8.7 (s, 1H); Mass Spectrum: M+H⁺ 423 and 425.

The 4-chloro-6-(3-chloropropoxy)-7-methoxyquinazoline used as a starting material was prepared as follows:-
Di-tert-butyl azodicarboxylate (1.84 g) was added portionwise over a few minutes to a stirred mixture of 4-chloro-6-hydroxy-7-methoxyquinazoline (1.2 g), 3-chloropropanol (0.572 ml), triphenylphosphine (2.1 g) and methylene chloride (30 ml) and the reaction mixture was stirred at ambient temperature for 3 hours. The mixture was evaporated and the residue was purified by column chromatography on silica using increasingly polar mixtures of methylene chloride and ethyl acetate as eluent. The material so obtained was triturated under diethyl ether. The resultant solid was isolated and dried under vacuum. There was thus obtained 4-chloro-6-(3-chloropropoxy)-7-methoxyquinazoline as a white solid (0.84 g); NMR Spectrum: (CDCl₃) 2.4 (m, 2H), 3.8 (t, 2H), 4.05 (s, 3H), 4.35 (t, 2H), 7.35 (s, 1H), 7.45 (s, 1H), 8.9 (s, 1H).

### Example 9

### 4-(5-chloro-2,3-methylenedioxypyrid-4-ylamino)-7-(3-chloroprnpoxy)-5-tetrahydropyran-4-yloxyquinazoline

Using an analogous procedure to that described in Example 1, 4-chloro-7-(3-chloropropoxy)-S-tetrahydropyran-4-yloxyquinazoline was reacted with 4-amino-5-chloro-2,3-methylenedioxypyridine to give the title compound in 78% yield; Mass Spectrum: M+H⁺ 493 and 495.

The 4-chloro-7-(3-chloropropoxy)-5-tetrahydropyran-4-yloxyquinazoline used as a starting material was prepared as follows:-
Using an analogous procedure to that described in the portion of Example 4 that is concerned with the preparation of starting materials, 4-chloro-7-hydroxy-5-tetrahydropyran-4-yloxyquinazoline (2.5 g) was reacted with 3-chloropropanol. There was thus obtained the required starting material in 21 % yield; NMR Spectrum: (DMSOd₆ and CF₃CO₂D) 1.7 (m, 2H), 2.0 (m, 2H), 2.25 (m, 2H), 3.55 (m, 2H), 3.8 (t, 2H), 3.9 (m, 2H), 4.3 (t, 2H), 4.95 (m, 1H), 6.8 (s, 1H), 6.9 (s, 1H), 9.2 (s, 1H).

### Example 10

### 4-(5-chloro-2,3-methylenedioxypyrid-4-ylamino)-7-(2,4-dimethoxybenzyloxy)-5-isopropoxyquinazoline

Using an analogous procedure to that described in Example 1, 4-chloro-7-(2,4-dimethoxybenzyloxy)-5-isopropoxyquinazoline was reacted with 4-amino-5-chloro-2,3-methylenedioxypyridine to give the title compound in 75% yield; NMR Spectrum: (CDCl₃) 1.55 (d, 6H), 3.8 (s, 3H), 3.85 (s, 3H), 4.8 (m, 1H), 5.15 (s, 2H), 6.15 (s, 2H), 6.5 (m, 2H), 6.6 (s, 1H), 7.0 (s, 1H), 7.35 (d, 1H), 7.75 (s, 1H), 8.6 (s, 1H), 9.6 (s, 1H); Mass Spectrum M+H⁺ 525 and 527.

The 4-chloro-7-(2,4-dimethoxybenzyloxy)-5-isopropoxyquinazoline used as a starting material was prepared as follows:-
Sodium hydride (60% dispersion in mineral oil; 40 g) was added portionwise to a solution of isopropanol (30 g) in DMF (500 ml) that had been cooled to 5°C. The mixture was allowed to warm to ambient temperature and was stirred for 60 minutes. 5,7-Difluoro-3,4-dihydroquinazolin-4-one (International Patent Application WO 01/94341; 90 g) was added and the mixture was stirred at ambient temperature for 3 hours. The mixture was poured into water (1 litre) and, with vigorous stirring, glacial acetic acid was added to acidify the mixture to pH5. The resultant solid was isolated, washed with water and with diethyl ether and dried under vacuum There was thus obtained 7-fluoro-5-isopropoxy-3,4-dihydroquinazolin-4-one (79 g); NMR Spectrum: (DMSOd₆) 1.31 (s, 6H), 4.73 (m, 1H), 6.89 (m, 1H), 6.95 (m, 1H), 7.96 (s, 1H); Mass Spectrum: M+H⁺ 223.

A mixture of 7-fluoro-5-isopropoxy-3,4-dihydroquinazolin-4-one (61 g), 2,4-dimethoxybenzyl alcohol (138 g), potassium tert-butoxide (185 g) and THF (1.5 litres) was stirred and heated to reflux for 18 hours. After cooling, the solvent was evaporated and a mixture of methylene chloride (400 ml) and water (600 ml) was added. With cooling, the 2-phase mixture was neutralised by the addition of 2N aqueous hydrochloric acid. The mixture was filtered and the organic phase was separated, dried over magnesium sulphate and evaporated. The residue was triturated under diethyl ether. There was thus obtained 7-(2,4-dimethoxybenzyloxy)-5-isopropoxy-3,4-dihydroquinazolin-4-one (68 g); NMR Spectrum: (DMSOd₆) 1.28 (s, 6H), 3.78 (s, 3H), 3.82 (s, 3H), 4.63 (m 1H), 5.06 (s, 2H), 6.55 (m, 2H), 6.62 (s, 1H), 6.71 (s, 1H), 7.33 (d, 1H), 7.88 (s, 1H); Mass Spectrum: M+H⁺ 371.

A mixture of a portion (4 g) of the material so obtained, phosphorus oxychloride (1.98 g), diisopropylethylanine (3.6 g) and methylene chloride (100 ml) was stirred and heated to 75°C for 3 hours. The mixture was cooled and evaporated. The residue was dried under vacuum for 1 hour and purified by column chromatography on silica using a 20:3 mixture of methylene chloride and ethyl acetate as eluent. There was thus obtained 4-chloro-7-(2,4-dimethoxybenzyloxy)-5 isopropoxyquinazoline as a solid (2.63 g); NMR Spectrum: (CDCl₃) 1.46 (s, 3H), 1.47 (s, 3H), 3.83 (s, 3H), 3.85 (s, 3H), 4.68 (m, 1H), 5.16 (s, 2H), 6.52 (m, 2H), 6.65 (s, 1H), 7.06 (s, 1H), 7.33 (d, 1H), 8.78 (s, 1H); Mass Spectrum: M+H⁺ 389.

### Example 11

### 4-(5-chloro-2,3-methylenedioxypyrid-4-ylamino)-7-hydroxy-5-isopropoxyquinazoline

Trifluoroacetic acid (4.5 ml) was added to a solution of 4-(5-chloro-2,3-methylenedioxypyrid-4-ylamino)-7-(2,4-dimethoxybenzyloxy)-5-isopropoxyquinazoline (0.53 g) in methylene chloride (9 ml) and the reaction mixture was stirred at ambient temperature for 30 minutes. The solvents were evaporated to give the di-trifluoroacetic acid salt (0.618 g) of the required compound. A portion of this salt was dissolved in methylene chloride (2 ml) and a 7M methanolic ammonia solution was added. The mixture was filtered and the filtrate was evaporated. There was thus obtained the title compound; Mass Spectrum: M+H⁺ 375 and 377.

### Example 12

### 4-(5-chloro-2,3-methylenedioxypyrid-4-ylamino)-7-(3-chloropropoxy)-5-isopropoxyquinazoline

A mixture of 4-(5-chloro-2,3-methylenedioxypyrid-4-ylamino)-7-hydroxy-5-isopropoxyquinazoline di-trifluoroacetic acid salt (0.615 g), 1 ,3-dichloropropane (0.38 ml), potassium carbonate (0.56 g) and DMF (6 ml) was stirred and heated to 80°C for 5 hours. After cooling, the solids were filtered off and the filtrate was evaporated. The residue was purified by column chromatography on silica using a 24:1 mixture of methylene chloride and methanol as eluent. There was thus obtained the title compound (0.32 g); NMR Spectrum: (CDCl₃) 1.55 (d, 6H), 2.3 (m, 2H), 3.8 (t, 2H), 4.25 (t, 2H), 4.9 (m, 1H), 6.15 (s, 2H), 6.5 (s, 1H), 6.9 (s, 1H), 7.75 (s, 1H), 8.6 (s, 1H), 9.6 (s, 1H).

### Example 13

### 4-(5-chloro-2,3-methylenedioxypyrid-4+ylamino)-7-(3chloropropoxy)quinazoline

Using an analogous procedure to that described in Example 1, 4-chloro-7-(3-chloropropoxy)quinazoline was reacted with 4-amino-5-chloro-2,3-methylenedioxypyridine to give the title compound in 89% yield; NMR Spectrum: (DMSOd₆ and CF₃CO₂D) 2.25 (m, 2H), 3.8 (t, 2H), 4.35 (t, 2H), 6.25 (s, 2H), 7.35 (s, 1H), 7.6 (d, 1H), 7.9 (s, 1H), 8.7 (d, 1H), 9.0 (s, 1H).

The 4-chloro-7-(3-chloropropoxy)quinazoline used as a starting material was prepared as follows:-
Sodium hydride (60% dispersion in mineral oil; 2.92 g) was added portionwise over 45 minutes to a stirred mixture of 1,3-propanediol (5.3 ml) and DMF (20 ml) that had been cooled to 0°C. The resultant mixture was stirred at ambient temperature for 1 hour and then heated to 60°C. 7-Fluoro-3,4-dihydroquinazolin-4-one (International Patent Application WO 01/04102, Example 2, Note [12] thereof; 2 g) was added and the reaction mixture was stirred and heated to 115°C for 3.5 hours. The reaction mixture was cooled to 0°C and water (50 ml) was added. The mixture was acidified to pH5.9 with 2N aqueous hydrochloric acid. The resultant precipitate was collected by filtration, washed with water and dried under vacuum over phosphorus pentoxide at 40°C. The solid so obtained was washed with diethyl ether and dried again under vacuum There was thus obtained 7-(3-hydroxypropoxy)-3,4-dihydroquinazolin-4-one (2.1 g); NMR Spectrum (DMSOd₆) 1.9 (m, 2H), 3.6 (m, 2H), 4.15 (m, 2H), 4.6 (br s, 2H), 7.1 (m, 2H), 8.05 (m, 2H); Mass Spectrum: M+H⁺221.

A mixture of 7-(3-hydroxypropoxy)-3,4-dihydroquinazolin-4-one (1 g), 1,2-dichloroethane (50 ml), triphenylphosphine (5.24 g) and carbon tetrachloride (2.9 ml) was stirred and heated to 70°C for 2 hours. The solvent was evaporated and the residue was purified by column chromatography on silica using initially methylene chloride followed by gradually increasing the polarity of the solvent up to a 9:1 mixture of methylene chloride and methanol as eluent. There was thus obtained 4-chloro-7-(3-chloropropoxy)quinazoline (1.23 g; containing 0.6 mole of triphenylphosphine oxide per mole of product); Mass Spectrum: M+H⁺ 393 and 395.

### Example 14

### 7-(2-chloroethoxy)-4-(2,3-methylenedioxypyrid-4-ylamino)-6-methoxyquinazotine

Sodium hexamethyldisilazane (1M solution in THF; 2 ml) was added dropwise to a mixture of 4-amino-2,3-methylenedioxypyridine (0.138 g), 4-chloro-7-(2-chloroethoxy)-6-methoxyquinazoline (0.272 g) and THF (5 ml) that had been cooled to 0°C. The mixture was stirred at 0°C for 1 hour. The resultant mixture was allowed to warm to ambient temperature and was stirred for 2 hours. The reaction was quenched by the addition of glacial acetic acid (0.12 ml). The solvents were evaporated and the residue was partitioned between methylene chloride and an aqueous ammonium hydroxide solution. The organic layer was collected and concentrated to a small volume. Diethyl ether was added and a precipitate formed. The resultant solid was isolated, washed with diethyl ether and dried. There was thus obtained the title compound (0.245 g); NMR Spectrum: (DMSOd₆) 3.97 (s, 3H), 4.04 (m, 2H), 4.45 (m, 2H), 6.12 (s, 2H), 7.13 (br d, 1H), 7.25 (s, 1H), 7.60 (d, 1H), 7.83 (s, 1H), 8.47 (s, 1H), 9.87 (br s, 1H); Mass Spectrum: M+H⁺ 375.

The 4-amino-2,3-methylenedioxypyridine used as a starting material was prepared as follows:-
Dibromomethane (31.5 ml) was added to a mixture 2,3-dihydroxypyridine (33 g), potassium carbonate (62 g) and NMP (200 ml) and the mixture was stirred and heated to 90°C for 16 hours. The mixture was cooled to ambient temperature and filtered. The filtrate was partitioned between diethyl ether (5 x 100 ml) and water (200 ml). The organic extracts were combined and concentrated under vacuum to a volume of about 20 ml. Petroleum ether (b.p 40-60°C; 300 ml) was added and the solution was washed with brine. The organic layer was separated and evaporated. There was thus obtained 2,3-methylenedioxypyridine as a liquid (5.1 g); NMR Spectrum: (CDCl₃) 6.05 (s, 2H), 6.76 (m, 1H), 6.99 (d, 1H), 7.65 (d, 1H).

Using an analogous procedure to that described in the second paragraph of the portion of Example 1 that is concerned with the preparation of the starting material 4-amino-5-chloro-2,3-methylenedioxypyridine, 2,3-methylenedioxypyridine was reacted with carbon dioxide gas to give 2,3-methylenedioxypyridine-4-carboxylic acid in 80% yield; NMR Spectrum (DMSOd₆) 6.24 (s, 2H), 7.13 (d, 1H); 7.63 (d, 1H).

Using an analogous procedure to that described in the third paragraph of that portion of Example 1 that is concerned with the preparation of starting materials, 2,3-methylenedioxypyridine-4-carboxylic acid was reacted with diphenylphosphoryl azide and anhydrous tert-butanol to give tert-butyl 2,3-methylenedioxypyrid-4-ylcarbamate in 62% yield; Mass Spectrum: M+H⁺ 239.

Using an analogous procedure to that described in the last paragraph of that portion of Example 1 that is concerned with the preparation of starting materials, tert-butyl 2,3-methylenedioxypyrid-4-ylcarbamate was reacted with trifluoroacetic acid to give 4-amino-2,3-methylenedioxypyridine in 80% yield; NMR Spectrum: (CDCl₃) 3.98 (m, 2H), 5.98 (s, 2H), 6.24 (d, 1H), 7.44 (d, 1H); Mass Spectrum: M+H⁺ 139.

### Example 15

### 7-(3-chloropropoxy)-4-(2,3-methylenedioxypyrid-4-ylamino)-6-methoxyquinazoline

Using an analogous procedure to that described in Example 14, 4-chloro-7-(3-chloropropoxy)-6-methoxyquinazoline was reacted with 4-amino-2,3-methylenedioxypyridine to give the title compound in 68% yield; NMR Spectrum: (DMSOd₆) 2.26 (m, 2H), 3.83 (m, 2H), 3.96 (s, 3H), 4.28 (m, 2H), 6.12 (s, 2H), 7.15 (br d, 1H), 7.25 (s, 1H), 7.61 (d, 1H), 7.81 (s, 1H), 8.49 (s, 1H), 9.79 (br s, 1H); Mass Spectrum: M+H⁺ 389.

### Example 16

### 7-[2-(4-acetylpiperazin-1-yl)ethoxy]-4-(2,3-methylenedioxypyrid-4-ylamino)-5-tetrahydropyran-4-yloxyquinazoline

Using an analogous procedure to that described in Example 1, 7-(2-(4-acetylpiperazin-1-yl)ethoxy]-4-chloro-5-tetrahydropyran-4-yloxyquinazoline (0.113 g) was reacted with 4-amino-2,3-methylenedioxypyridine (0.036 g). The reaction mixture was quenched with glacial acetic acid (0.031 g) and diluted with methanol. The mixture was evaporated and the residue was purified by column chromatography on a C 18 reversed phase silica column (Waters Symmetry column,5 microns silica, 20 mm diameter, 100 mm length) using a decreasingly polar mixture of water and acetonitrile (containing 1% acetic acid) as eluent. The material so obtained was diluted with a 7M methanolic ammonia solution. The mixture was evaporated and the material so obtained was dissolved in methylene chloride. The solution was dried over magnesium sulphate and evaporated to give the title compound as a foam in 53% yield; NMR Spectrum: (CDCl₃) 2.02 (m, 2H), 2.1 (s, 3H), 2.22 (m, 2H), 2.6 (m, 4H), 2.9 (m, 2H), 3.5 1 (m, 2H), 3.6 (m, 2H), 3.66 (m, 2H), 4.1 (m, 2H), 4.25 (m, 2H), 4.73 (m, 1H), 6.13 (s, 2H), 6.59 (s, 1H), 6.9 (s, 1H), 7.7 (d, 1H), 8.36 (d, 1H), 8.66 (s, 1H); Mass Spectrum: M+H⁺ 537.

The 7-[2-(4-acetylpiperazin-1-yl)ethoxy]-4-chloro-5-tetrahydropyran-4-yloxyquinazoline used as a starting material was prepared as follows:-
Sodium hydride (60% dispersion in mineral oil; 0.6 g) was added portionwise to a solution of 4-hydroxytetrahydropyran (0.78 g) in DMF (10 ml) that had been cooled to 5°C. The mixture was allowed to warm to ambient temperature and was stirred for 15 minutes. 5,7-Difluoro-3,4-dihydroquinazolin-4-one (International Patent Application WO 01/94341; 0.9 g) was added and the mixture was stirred at ambient temperature for 30 minutes. The mixture was poured into water (100 ml) and, with vigorous stirring, glacial acetic acid was added to acidify the mixture to pH5. The resultant solid was isolated, washed with water and with diethyl ether and dried under vacuum. There was thus obtained 7-fluoro-5-tetrahydropyran-4-yloxy-3,4-dihydroquinazolin-4-one (1.1 g); NMR Spectrum: (DMSOd₆) 1.6-1.75 (m, 2H), 1.9-2.0 (m, 2H), 3.5-3.6 (m, 2H), 3.85-3.95 (m, 2H), 4.8 (m, 1H), 6.9 (m, 1H), 7.05 (m, 1H), 8.0 (s, 1H); Mass Spectrum: M+H⁺265.

After repetition of the prior reaction, a mixture of 7-fluoro-S-tetrahydropyran-4-yloxy-3,4-dihydroquinazolin-4-one (5.3 g), 2-piperazin-1-ylethanol (3.9 g), potassium tert-butoxide (6.7 g) and THF (200 ml) was stirred and heated to reflux for 3 hours. A second portion (6.7 g) of potassium tert-butoxide was added and the mixture was heated to reflux for a further 12 hours. The mixture was cooled to ambient temperature and filtered. The filtrate was evaporated and the residue was purified by column chromatography on silica using increasingly polar mixtures of methylene chloride and a 7M methanolic ammonia solution as eluent. The material so obtained was triturated under diethyl ether. There was thus obtained 7-(2-piperazin-1-ylethoxy)-5-tetrahydropyran-4-yloxy-3,4-dihydroquinazolin-4-one (5.2 g); NMR Spectrum: (DMSOd₆ and CF₃CO₂D) 1.75 (m, 2H), 2.03 (m, 2H), 3.2-4.0 (m, 14H), 4.59 (m, 2H), 4.92 (m, 1H), 6.88 (s, 1H), 6.9 (s, 1H), 9.28 (s, 1H); Mass Spectrum: M+H⁺ 375.

Acetic anhydride (1.5 1 ml) was added dropwise to a stirred mixture of 7-(2-piperazin-1-ylethoxy)-5-tetrahydropyran-4-yloxy-3,4-dihydroquinazolin-4-one (5 g) and water (20 ml) and the resultant mixture was stirred at ambient temperature for 10 minutes. The reaction mixture was evaporated and the residue was triturated under diethyl ether. The resultant solid was isolated, washed with diethyl ether and dried under vacuum There was thus obtained 7-[2-(4-acetylpiperazin-1-yl)ethoxy]-5-tetrahydropyran-4-yloxy-3,4-dihydroquinazolin-4-one (5.5 g); NMR Spectrum: (DMSOd₆ and CF₃CO₂D) 1.75 (m, 2H), 2.03 (m, 2H), 2.08 (s, 3H), 3.0-4.2 (m, 13H), 4.56 (m, 3H), 4.94 (m, 1H), 6.84 (s, 1H), 6.9 (s, 1H), 9.21 (s, 1H); Mass Spectrum: M+H⁺ 417.

A mixture of a portion (0.416 g) of the material so obtained, triphenylphosphine (0.655 g), carbon tetrachloride (0.34 ml) and 1,2-dichloroethane (20 ml) was stirred and heated to 70°C for 1.5 hours. The mixture was evaporated and the residue was purified by column chromatography on silica using increasingly polar mixtures of methylene chloride and a 7M methanolic ammonia solution (a solvent gradient having from 1% to 3% methanolic ammonia solution) as eluent. There was thus obtained 7-[2-(4-acetylpiperazin-1-yl)ethoxy]-4-chloro-5-tetrahydropyran-4-yloxyquinazoline as a solid (0.35 g); NMR Spectrum: (CDCl₃) 2.0 (m, 2H), 2.1 (s, 3H), 2.12 (m, 2H), 2.58 (m, 4H), 2.9 (m, 2H), 3.51 (m, 2H), 3.68 (m, 4H), 4.05 (m, 2H), 4.25 (m, 2H), 4.75 (m, 1H), 6.62 (s, 1H), 6.94 (s, 1H), 8.82 (s, 1H); Mass Spectrum: M+H⁺ 435 and 437.

### Example 17

### 7-[2-(4-acetylpiperazin-1-yl)ethoxy]-4-(2,3-methylenedioxypyrid-4-ylamino)-5-isopropoxyquinazoline

Using an analogous procedure to that described in Example 16, 7-[2-(4-acetylpiperazin-1-yl)ethoxy]-4-chloro-5-isopropoxyquinazoline was reacted with 4-amino-2,3-methylenedioxypyridine to give the title compound in 55% yield; NMR Spectrum: (CDCl₃) 1.55 (s, 3H), 1.56 (s, 3H), 2.1 (s, 3H), 2.59 (m, 4H), 2.89 (m, 2H), 3.51 (m, 2H), 3.67 (m, 2H), 4.24 (m 2H), 4.85 (m, 1H), 6.13 (s, 2H), 6.57 (s, 1H), 6.85 (s, 1H), 7.71 (d, 1H), 8.41 (d, 1H), 8.66 (s, 1H); Mass Spectrum: M+H⁺ 495.

The 7-[2-(4-acetylpiperazin-1-yl)ethoxy]-4-chloro-5-isopropoxyquinazoline that is required as a starting material was prepared as follows using analogous procedures to those described in the portion of Example 16 that is concerned with the preparation of starting materials.

5,7-Difluoro-3,4-dihydroquinazolin-4-one was reacted with isopropanol to give 7-fluoro-5-isopropoxy-3,4-dihydroquinazolin-4-one in 73% yield; NMR Spectrum: (DMSOd₆) 1.31 (s, 6H), 4.73 (m, 1H), 6.89 (m, 1H), 6.95 (m, 1H), 7.96 (s, 1H); Mass Spectrum: M+H⁺ 223.

The material so obtained was reacted with 2-piperazin-1-ylethanol to give 5-isopropoxy-7-(2-piperazin-1-ylethoxy)-3,4-dihydroquinazolin-4-one in 63% yield; NMR Spectrum: (CDCl₃) 1.45 (s, 3H), 1.46 (s, 3H), 2.4-3.0 (m, 10H), 4.2 (t, 2H), 4.62 (m, 1H), 6.51 (s, 1H), 6.72 (s, 1H), 7.9 (s, 1H).

The material so obtained was reacted with an excess of acetic anhydride but using methylene chloride rather than water as the reaction solvent. The reaction mixture was stirred at ambient temperature for 15 minutes. The mixture was partitioned between methylene chloride and a saturated aqueous sodium bicarbonate solution. The organic layer was washed with water and with brine, dried over magnesium sulphate and evaporated. The residue was triturated under a mixture of acetonitrile and diethyl ether. There was thus obtained 7-[2-(4-acetylpiperazin-1-yl)ethoxy]-5-isopropoxy-3,4-dihydroquinazolin-4-one in 70% yield; NMR Spectrum: (CDCl₃) 1.46 (s, 3H), 1.47 (s, 3H), 2.1 (s, 3H), 2.58 (m, 4H), 2.87 (t, 2H), 3.5 (m, 2H), 3.66 (m, 2H), 4.21 (t, 2H), 4.63 (m, 1H), 6.51 (s, 1H), 6.72 (s, 1H), 7.9 (s, 1H), 9.9 (br s, 1H); Mass Spectrum: M+H⁺ 375.

The material so obtained was reacted with carbon tetrachloride and triphenylphosphine to give 7-[2-(4-acetylpiperazin-1-yl)ethoxy]-4-chloro-5-isopropoxyquinazoline in 68% yield which was used without further purification.

### Example 18

### 4-(5-chloro-2,3-methylenedioxypyrid-4-ylamino)-7-{2-[4-(2-dimethylaminoacetyl)piperazin-1-yl]ethoxy}-5-isopropoxyquinazoline

4-(5-Chloro-2,3-methylenedioxypyrid-4-ylamino)-5-isopropoxy-7-(2-piperazin-1-ylethoxy)quinazoline (0.2 g) was added to a stirred mixture of 2-dimethylaminoacetyl chloride hydrochloride (0.097 g), triethylamine (0.15 ml) and methylene chloride (5 ml) that had been cooled to 0°C. The reaction mixture was allowed to warm to ambient temperature and stirred for 2 hours. A second portion of each of 2-dimethylaminoacetyl chloride hydrochloride (0.097 g) and triethylamine (0.057 ml) were added and the reaction was stirred at ambient temperature for 16 hours overnight. Methylene chloride (50 ml) was added and the reaction mixture was extracted twice with a saturated aqueous sodium bicarbonate solution. The organic phase was dried over magnesium sulphate and evaporated. The residue was purified by column chromatography on silica using increasingly polar solvent mixtures, starting with a 9:1 mixture of methylene chloride and methanol and ending with a 90:8:2 mixture of methylene chloride, methanol and a saturated methanolic ammonia solution. There was thus obtained the title compound as a foam (0.155 g); NMR Spectrum: (CDCl₃) 1.55 (d, 6H), 2.3 (s, 6H), 2.6 (m, 4H), 2.9 (t, 2H), 3.1 (s, 2H), 3.65 (m, 4H), 4.25 (t, 2H), 4.85 (s, 1H), 6.15 (s, 2H), 6.55 (s, 1H), 6.85 (s, 1H), 7.75 (s, 1H), 8.6 (s, 1H), 9.6 (s, 1H); Mass Spectrum: M+H⁺ 572 and 574; Elemental Analysis: Found C, 55.1; H, 6.1; N, 16.8;
C₂₇H₃₄ClN₇O₅ 0.75H₂O requires C, 55.4; H, 6.1; N, 16.7%.

### Example 19

### 7-(N-tert-butoxycarbonylpiperidin-4-ylmethoxy)-4-(5-chloro-2,3-methylenedioxypyrid-4-ylamino)-6-methoxyquinazoline

Using a similar procedure to that described in Example 1, a solution of 4-amino-5-chloro-2,3-methylenedioxypyridine (0.193 g) in DMF (2 ml) was added to a stirred suspension of sodium hydride (60% dispersion in mineral oil, 0.048 g) in DMF (2 ml) and the mixture was stirred at ambient temperature for 15 minutes. A solution of 7-(N-tert-butoxycarbonylpiperidin-4-ylmethoxy)-4-chloro-6-methoxyquinazoline [International Patent Application WO 02/16352 (Note [24] within Example 2 thereof; 0.38 g] in DMF (4 ml) was added and the resultant mixture was stirred at ambient temperature for 1 hour. The reaction mixture was partitioned between ethyl acetate and brine. The organic phase was dried over magnesium sulphate and evaporated. The residue was purified by column chromatography on silica using a 49:1 mixture of methylene chloride and methanol. There was thus obtained the title compound as a solid (0.24 g); NMR Spectrum: (DMSOd₆) 1.29 (m, 2H), 1.45 (s, 9H), 1.8 (m, 2H), 2.04 (m, 1H), 2.83 (m, 2H), 4.0 (m, 7H), 8.12 (br s, 2H), 7.17 (br s, 1H), 7.72 (m, 2H), 8.37 (br s, 1H), 9.37 (br s, 1H); Mass Spectrum: M+H⁺ 544 and 546.

### Example 20

### 4-(5-chloro-2,3-methylenedioxypyrid-4-ylamino)-6-methoxy-7-(piperidin-4-ylmethoxy)quinazoline

Trifluoroacetic acid (1 ml) was added to a solution of 7-(N-tert-butoxycarbonylpiperidin-4-ylmethoxy)-4-(5-chloro-2,3-methylenedioxypyrid-4-ylamino)-6-methoxyquinazoline (0.253 g) in methylene chloride (10 ml) and the reaction mixture was stirred at ambient temperature for 1 hour. The reaction mixture was evaporated. Toluene was added to the residue and the mixture was evaporated. The residue was purified by column chromatography on silica (Isolute SCX column) using a 7M methanolic ammonia solution as eluent. There was thus obtained the title compound as a solid (0.187 g); NMR Spectrum: (DMSOd₆) 1.25 (m, 2H), 1.75 (d, 2H), 1.93 (m, 1H), 2.54 (m, 2H), 3.0 (d, 2H), 3.93 (s, 3H), 3.98 (d, 2H), 6.17 (s, 2H), 7.15 (s, 1H), 7.76 (s, 1H), 7.78 (s, 1H), 8.23 (s, 1H); Mass Spectrum: M+H⁺ 444 and 446.

### Example 21

### 4-(5-chloro-2,3-methylenedioxypyrid-4-ylamino)-7-[N-(2-dimethylaminoacetyl)piperidin-4-ylmethoxy]-6-methoxyquinazoline

Diisopropylethylamine (0.118 ml) was added to a mixture of 4-(5-chloro-2,3-methylenedioxypyrid-4-ylamino)-6-methoxy-7-(piperidin-4-ylmethoxy)quinazoline (0.15 g), N,N-dimethylglycine (0.042 g), 2-(7-azabenzotriazol-1-yl)-1,1,3,3-tetramethyluronium hexafluorophosphate(V) (0.154 g) and DMF (3 ml) and the reaction mixture was stirred at ambient temperature for 16 hours. The mixture was diluted with ethyl acetate and washed with brine. The organic solution was dried over magnesium sulphate and evaporated. The residue was purified by column chromatography on silica using a 100:3 mixture of methylene chloride and a 7M methanolic ammonia solution as eluent. There was thus obtained the title compound as a solid (0.051 g); NMR Spectrum: (DMSOd₆) 1.11-1.36 (m, 2H), 1.83 (d, 2H), 2.11 (m, 1H), 2.19 (s, 6H), 2.61 (t, 1H), 3.03 (m, 2H), 3.12 (d, 1H), 3.93 (s, 3H), 4.06 (m, 3H), 4.4 (d, 1H), 6.19 (br s, 2H), 7.19 (br s, 1H), 7.78 (m, 2H), 8.39 (br s, 1H), 9.71 (br s, 1H); Mass Spectrum: M+H⁺ 529 and 531.

### Example 22

### 7-[2-(4-acetylpiperazin-1-yl)ethoxy]-4-(5-chloro-2,3-methylenedioxypyrid-4-ylamino)-5-isopropoxyquinazoline

A mixture of 7-(2-chloroethoxy)-4-(5-chloro-2,3-methylenedioxypyrid-4-ylamino)-5-isopropoxyquinazoline (24 g), 1-acetylpiperazine (21 g), potassium iodide (18 g) and DMA (500 ml) was stirred and heated to 100°C for 4 hours. The solvent was evaporated and the residue was partitioned between methylene chloride (1 litre) and water (500 ml). The aqueous layer was extracted with methylene chloride. The organic solutions were combined, washed with brine, dried over magnesium sulphate and evaporated. The residue was purified by column chromatography on silica using increasingly polar mixtures of methylene chloride and methanol (from a 20:1 mixture to a 10:1 mixture) as eluent. After evaporation of the solvent, the material so obtained was triturated under diethyl ether. There was thus obtained the title compound as a white solid (26.2 g); m.p. 208-210°C.

The 7-(2-chloroethoxy)-4-(5-chloro-2,3-methylenedioxypyrid-4-ylamino)-5-isopropoxyquinazoline used as a starting material was obtained as follows:-
Sodium hexamethyldisilazane (1M solution in THF, 164 ml) was added dropwise over one hour to a ice-cooled mixture of 4-chloro-7-(2,4-dimethoxybenzyloxy)-5-isopropoxyquinazoline (32 g), 4-amino-5-chloro-2,3-methylenedioxypyridine (15.6 g) and THF (430 ml) whilst maintaining the temperature of the reaction mixture at about 3°C. At the end of the addition, the reaction mixture was allowed to warm to ambient temperature and was stirred for 2.5 hours. The reaction mixture was cooled to 0°C and a mixture of acetic acid (9.4 ml) and water (250 ml) was added. The mixture was evaporated and the residue was partitioned between methylene chloride and water, the basicity of the aqueous phase having been adjusted to 7.5 by the addition of 3N aqueous hydrochloric acid solution. The organic phase was separated and the aqueous phase was extracted three times with methylene chloride. The organic layers were combined, washed with brine, dried over magnesium sulphate and evaporated. The resultant solid was triturated under ethyl acetate. There was thus obtained 4-(5-chloro-2,3-methylenedioxypyrid-4-ylamino)-7-(2,4-dimethoxybenzyloxy)-5-isopropoxyquinazoline as a white solid (38 g); Mass Spectrum: M+H⁺ 525 and 527.
Triethylsilane (70 ml) and trifluoroacetic acid (48 ml) were added in turn to an ice-cooled solution of 4-(5-chloro-2,3-methylenedioxypyrid-4-ylamino)-7-(2,4-dimethoxybenzyloxy)-5-isopropoxyquinazoline (37.7 g) in methylene chloride (560 ml) and the resultant reaction mixture was stirred at ambient temperature for 1 hour. The solvents were evaporated under high vacuum The resultant solid was triturated under ethyl acetate. The material so obtained was isolated, washed with ethyl acetate and dried under high vacuum. There was thus obtained the di-trifluoroacetic acid salt (37.4 g) of 4-(5-chloro-2,3-methylenedioxypyrid-4-ylamino)-7-hydroxy-5-isopropoxyquinazoline which was used without further purification.

Potassium carbonate (34.6 g) was added to a mixture of 4-(5-chloro-2,3-methylenedioxypyrid-4-ylamino)-7-hydroxy-5-isopropoxyquinazoline di-trifluoroacetic acid salt (49 g), 1,2-dichloroethane (440 ml) and DMF (245 ml) and the mixture was stirred and heated to 90°C for 3.5 hours. An additional portion (7 g) of potassium carbonate was added and the mixture was stirred at 90°C for a further hour. The reaction mixture was cooled to ambient temperature and the solids were filtered off and washed with methylene chloride. The filtrate and washings were combined and evaporated. The resultant residue was purified by column chromatography on silica using increasingly polar mixtures of methylene chloride and methanol (from a 50:1 mixture to a 20:1 mixture) as eluent. There was thus obtained 7-(2-chloroethoxy)-4-(5-chloro-2,3-methylenedioxypyrid-4-ylamino)-5-isopropoxyquinazoline as a white solid (37.1 g); Mass Spectrum: M+H⁺ 437 and 439.

## Claims

1. A combination comprising an inhibitor of the Src family of non-receptor tyrosine kinases, or a pharmaceutically-acceptable salt thereof, wherein the Src inhibitor is:-4-(2-chloro-5-methoxyanilino)-6-methoxy-7-(N-methylpiperidin-4-ylmethoxy)quinazoline, or a pharmaceutically-acceptable acid-addition salt thereof, or 4-(6-chloro-2,3-methylenedioxyanilino )-7-[2-(4-methylpiperazin-1-yl)ethoxy]-5-tetrahydropyran-4-yloxyquinazoline, or a pharmaceutically-acceptable acid-addition salt thereof, and gemcitabine for use in the synergistic treatment or prophylaxis of pancreatic cancer.

2. A combination as claimed in claim 1 wherein the Src inhibitor is:-4-(6-chloro-2,3-methylenedioxyamlino)-7-[2-(4-methylpiperazin-1-yl)ethoxy]-5-tetrahydropyran-4-yloxyquinazoline; or a pharmaceutically-acceptable acid-addition salt thereof.

3. A combination as claimed in claim 1 wherein the Src inhibitor is:- 4-(2-chloro-5-methoxyanilino)-6-methoxy-7-(N-methylpiperidin-4-ylmethoxy)quinazoline; or a pharmaceutically-acceptable acid-addition salt thereof.

4. A pharmaceutical composition for use in the synergistic treatment or prophylaxis of pancreatic cancer which comprises a combination as defined in claim 1 in association with a pharmaceutically-acceptable excipient or carrier.

5. The use of a combination as defined in claim 1 in the manufacture of a medicament for administration to a warm-blooded animal such as man to provide the synergistic treatment or prophylaxis of pancreatic cancer.

## Patentansprüche

1. Kombination, enthaltend einen Inhibitor der Src-Familie von Nicht-Rezeptor-Tyrosinkinasen oder ein pharmazeutisch annehmbares Salz davon, worin es sich bei dem Src-Inhibitor um 4-(2-Chlor-5-methoxyanilino)-6-methoxy-7-(N-methylpiperidin-4-ylmethoxy)chinazolin oder ein pharmazeutisch annehmbares Säureadditionssalz davon oder 4-(6-Chlor-2,3-methylendioxyanilino)-7-[2-(4-methylpiperazin-1-yl)ethoxy]-5-tetrahydropyran-4-yloxy-chinazolin oder ein pharmazeutisch annehmbares Säureadditionssalz davon handelt, und Gemcitabin, zur Verwendung bei der synergistischen Behandlung oder Prophylaxe von Bauchspeicheldrüsenkrebs.

2. Kombination nach Anspruch 1, worin es sich bei dem Src-Inhibitor um 4-(6-Chlor-2,3-methylendioxy-anilino)-7-[2-(4-methylpiperazin-1-yl)ethoxy]-5-tetrahydropyran-4-yloxychinazolin oder ein pharmazeutisch annehmbares Säureadditionssalz davon handelt.

3. Kombination nach Anspruch 1, worin es sich bei dem Src-Inhibitor um 4-(2-Chlor-5-methoxyanilino)-6-methoxy-7-(N-methylpiperidin-4-ylmethoxy)-chinazolin oder ein pharmazeutisch annehmbares Säureadditionssalz davon handelt.

4. Pharmazeutische Zusammensetzung zur Verwendung bei der synergistischen Behandlung oder Prophylaxe von Bauchspeicheldrüsenkrebs, die eine Kombination gemäß Anspruch 1 zusammen mit einem pharmazeutisch annehmbaren Hilfsstoff oder Träger enthält.

5. Verwendung einer Kombination gemäß Anspruch 1 zur Herstellung eines Arzneimittels zur Verabreichung an einen Warmblüter wie den Menschen zur Bereitstellung der synergistischen Behandlung oder Prophylaxe von Bauchspeicheldrüsenkrebs.

## Revendications

1. Combinaison comprenant un inhibiteur de la famille Src de tyrosines kinases non récepteurs, ou un sel pharmaceutiquement acceptable de celui-ci, l'inhibiteur de Src étant : la 4-(2-chloro-5-méthoxyanilino)-6-méthoxy-7-(N-méthylpipéridin-4-ylméthoxy)quinazoline ou un sel d'addition d'acide pharmaceutiquement acceptable de celle-ci, ou la 4-(6-chloro-2,3-méthylènedioxyanilino)-7-[2-(4-méthylpipérazin-1-yl)-éthoxy]-5-tétrahydropyran-4-yloxyquinazoline ou un sel d'addition d'acide pharmaceutiquement acceptable de celle-ci, et la gemcitabine, destinée à être utilisée dans le traitement ou la prophylaxie synergique du cancer pancréatique.

2. Combinaison selon la revendication 1, **caractérisée en ce que** l'inhibiteur de Src est :
la 4-(6-chloro-2,3-méthylènedioxyanilino)-7-[2-(4-méthylpipérazin-1-yl)éthoxy]-5-tétrahydropyran-4-yloxyquinazoline ou un sel d'addition d'acide pharmaceutiquement acceptable de celle-ci.

3. Combinaison selon la revendication 1, **caractérisée en ce que** l'inhibiteur de Src est :
1 a 4-(2-chloro-5-méthoxyanilino)-6-méthoxy-7-(N-méthylpipéridin-4-ylméthoxy)quinazoline ou un sel d'addition d'acide pharmaceutiquement acceptable de celle-ci.

4. Composition pharmaceutique destinée à être utilisée dans le traitement ou la prophylaxie synergique du cancer pancréatique, **caractérisée en ce qu'**elle comprend une combinaison telle que définie dans la revendication 1 en association avec un excipient ou un support pharmaceutiquement acceptable.

5. Utilisation d'une combinaison telle que définie dans la revendication 1, dans la fabrication d'un médicament destiné à être administré à un animal à sang chaud tel que l'homme pour assurer le traitement ou la prophylaxie synergique du cancer pancréatique.
